# EUROPEAN PATENT APPLICATION

(11) **EP 4 383 987 A1**
(43) Date of publication of application: **12.06.2024**
(21) Application number: 22211662.6
(22) Date of filing: 06.12.2022
(51) Int. Cl.: H10K 85/60, H10K 50/16

(54) **ORGANIC LIGHT EMITTING DIODE, DEVICE COMPRISING THE SAME AS AND COMPOUND FOR USE THEREIN**

(71) Applicant: Novaled GmbH, 01099 Dresden (DE)
(72) Inventor: Elena, GALÁN GARCÍA c/o Novaled GmbH, 01099 Dresden (DE); Yevhen, KARPOV c/o Novaled GmbH, 01099 Dresden (DE)
(74) Representative: Bittner, Thomas L.

(57) **Abstract**

The present invention relates to an organic light emitting diode and a device comprising the same. The present invention further relates to a compound suitable for use in the organic light emitting diode.

## Description

### TECHNICAL FIELD

The present invention relates to an organic light emitting diode and a device comprising the same. The present invention further relates to a compound suitable for use in the organic light emitting diode.

### BACKROUND OF THE INVENTION

Organic semiconducting devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic semiconducting device, such as an organic light emitting diode, may be applied to a large-size flat panel display.

It is, therefore, the object of the present invention to provide organic light emitting diodes and compounds for preparing the same overcoming drawbacks of the prior art, in particular providing compounds for use in organic light emitting diodes comprising the same helpful to improve the performance thereof, especially with respect to lifetime and voltage rise over time.

### DISCLOSURE

The object is achieved by an organic light emitting diode, comprising a non-transparent substrate, an anode, a cathode, an emission layer, an electron injection layer and an electron transport layer stack;
wherein
- the electron transport layer stack is arranged between the emission layer and the electron injection layer;
- the electron transport layer stack comprises a first electron transport layer and a second electron transport layer;
- the first electron transport layer is in direct contact with the emission layer;
- the first electron transport layer comprises a compound of Formula (I) 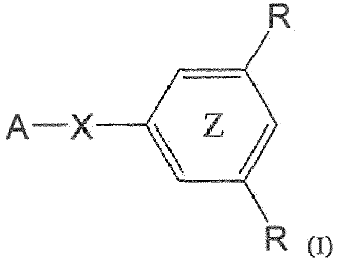 wherein
- A is an unsubstituted or substituted C₃ to C₂₁ heteroaryl comprising at least one six-membered ring, wherein the six-membered ring comprises at least one N-atom;
   - wherein the substituted C₃ to C₂₁ heteroaryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R is independently selected from the group consisting of substituted or unsubstituted C₁₀ to C₁₈ aryl and a substituted or unsubstituted group of the following formula (2); 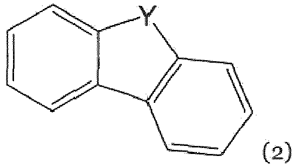
   - wherein Y is independently selected from the group consisting of CR'₂, SiR'₂, S and O, wherein R' is independently selected from the group consisting of C₁ to C₁₂ alkyl and C₆ to C₁₈ aryl;
   - wherein the substituted C₁₀ to C₁₈ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
   - wherein the substituted group of the following formula (2) is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R comprises two 6-membered aryl rings;
- R is bonded to the ring Z by a first C-atom of one of the two 6-membered aryl rings; and the first C-atom is adjacent to a second C-atom, wherein the second C-atom is a sp²-C-atom and the second C-atom is not bonded to H;
- X is a single bond or substituted or unsubstituted C₆ to C₆₀ aryl;
   - wherein the substituted C₆ to C₆₀ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- X is bonded to a C-atom of the six-membered ring comprising at least one N-atom of A; and
- the second electron transport layer does not comprise a compound according to formula (I).

The object is further achieved by a display device comprising the organic light emitting diode according to the present invention.

The object is further achieved by a compound of Formula (II) 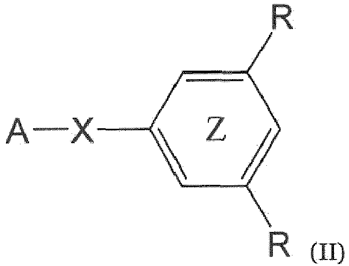 wherein
- A is an unsubstituted or substituted C₃ to C₂₁ heteroaryl comprising at least one six-membered ring, wherein the six-membered ring comprises at least two N-atoms;
   - wherein the substituted C₃ to C₂₁ heteroaryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R is independently selected from the group consisting of substituted or unsubstituted C₁₀ to C₁₈ aryl and a substituted or unsubstituted group of the following formula (2) 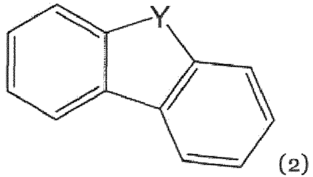
   - wherein Y is independently selected from the group consisting of CR'₂, SiR'₂, S and O, wherein R' is independently selected from the group consisting of C₁ to C₁₂ alkyl and C₆ to C₁₈ aryl;
   - wherein the substituted C₁₀ to C₁₈ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₃ to C₇ alkyl, perfluorinated C₁ to C₇ alkyl, partially fluorinated C₁ to C₇ alkoxy, perfluorinated C₁ to C₇ alkoxy, D, F and CN;
   - wherein the substituted group of the following formula (2) is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R comprises two 6-membered aryl rings;
- R is bonded to the phenylene-ring Z by a first C-atom of one of the two 6-membered aryl rings; and the first C-atom is adjacent to a second C-atom, wherein the second C-atom is an sp²-C-atom and the second C-atom is not bonded to H;
- X is a single bond or C₆ to C₆₀ aryl;
   - wherein the substituted C₆ to C₆₀ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- X is bonded to a C-atom of the six-membered ring comprising at least two N-atoms of A.

### Compound of formula (I)

The first electron transport layer comprises a compound of formula (I)

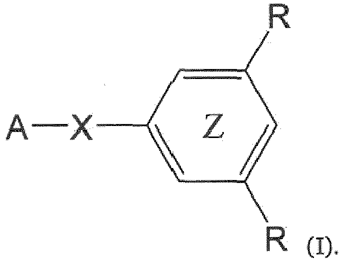

In the formula (I), the "Z" in the phenylene ring has no chemical meaning but merely serves to facilitate reference to the ring in the present disclosure. Whenever reference to the "ring Z" is made herein, reference is made to the phenylene ring connecting A-X- and the two R.

A is an unsubstituted or substituted C₃ to C₂₁ heteroaryl comprising at least one six-membered ring, wherein the six-membered ring comprises at least one N-atom. A may be an unsubstituted or substituted C₃ to C₂₁ heteroaryl comprising at least one six-membered ring, wherein the six-membered ring comprises at least two N-atoms. A may be an unsubstituted or substituted C₃ to C₂₁ heteroaryl comprising at least one six-membered ring, wherein the six-membered ring comprises three N-atoms. A may be an unsubstituted or substituted C₃ to C₂₁ heteroaryl comprising at least one six-membered ring, wherein the six-membered ring is a triazine ring.

A may be an unsubstituted or substituted C₃ to C₁₅ heteroaryl comprising at least one six-membered ring, wherein the six-membered ring comprises at least one N-atom. A may be an unsubstituted or substituted C₃ to C₁₅ heteroaryl comprising at least one six-membered ring, wherein the six-membered ring comprises at least two N-atoms. A may be an unsubstituted or substituted C₃ to C₁₅ heteroaryl comprising at least one six-membered ring, wherein the six-membered ring comprises three N-atoms. A may be an unsubstituted or substituted C₃ to C₁₅ heteroaryl comprising at least one six-membered ring, wherein the six-membered ring is a triazine ring.

A may have one of the following formulas 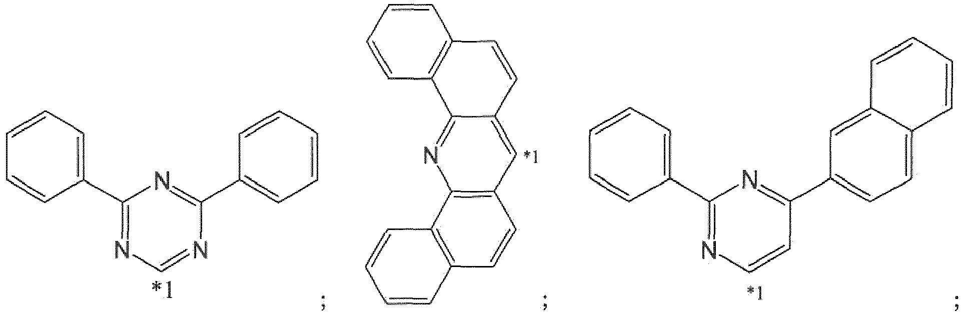 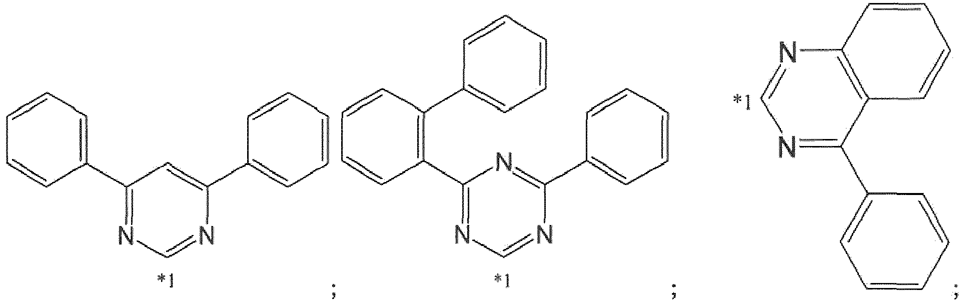 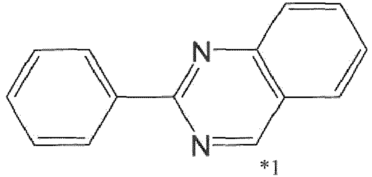 wherein A is bonded at ^{∗}1 to X.

A may have the following formula (Ia) 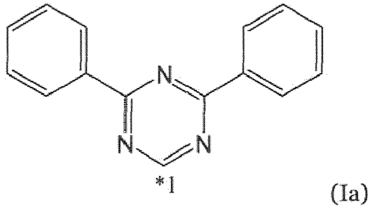 wherein Ia is bonded at ^{∗}1 to X.

X is bonded to a C-atom of the six-membered ring comprising at least one N-atom of A. For example, if A has the following formula 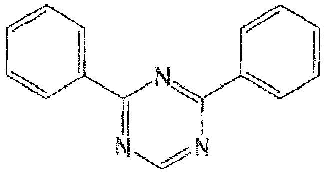 then binding to X is necessarily at the last remaining C-atom of the N-containing ring to form the following structure

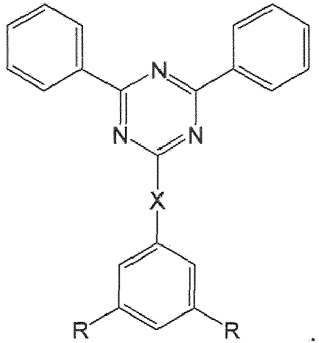

X is a single bond or substituted or unsubstituted C₆ to C₆, aryl. X may be a single bond or substituted or unsubstituted C₆ to C₅₄ aryl. X may be a single bond or substituted or unsubstituted C₆ to C₄₈ aryl. X may be a single bond or substituted or unsubstituted C₆ to C₄₂ aryl. X may be a single bond or substituted or unsubstituted C₆ to C₃₆ aryl. X may be a single bond or substituted or unsubstituted C₆ to C₃₀ aryl. X may be a single bond or substituted or unsubstituted C₆ to C₂₄ aryl. X may be a single bond or substituted or unsubstituted C₆ to C₁₈ aryl. X may be a single bond or substituted or unsubstituted C₆ to G₁₂ aryl.

X may be selected from a single bond or from the following formulas 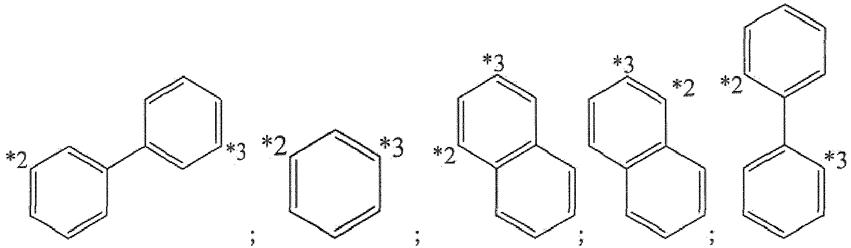 wherein X is bonded to A at ^{∗}2 and is bonded to the ring Z at ^{∗}3.

X may be selected from the structures (Ib) and (Ic) 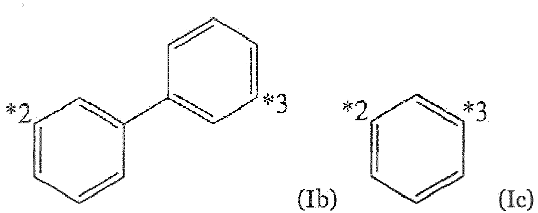 wherein Ib and Ic are bonded to A at ^{∗}2 and are bonded to the ring Z at ^{∗}3.

R comprises two 6-membered aryl rings. This applies for both R comprised in the compound of formula (I), respectively.

R is bonded to the (phenylene-)ring Z by a first C-atom of one of the two 6-membered aryl rings; and the first C-atom is adjacent to a second C-atom, wherein the second C-atom is a sp²-C-atom and the second C-atom is not bonded to H. This applies for both R comprised in the compound of formula (I), respectively. For example, if R has the following structure 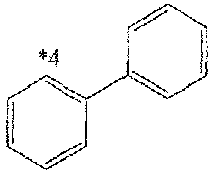 than the carbon atom marked with ^{∗}4^{∗} is the first C-atom and the adjacent carbon atom to which the phenyl-group is bonded is the second C-atom. In another example, if R has the following structure 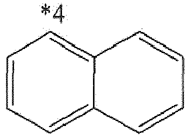 than the carbon atom marked with ^{∗}4 is the first C-atom and the adjacent bridgehead atom (at which the second ring is condensed) is the second C-atom.

R is independently selected from the group consisting of substituted or unsubstituted C₁₀ to C₁₈ aryl and a substituted or unsubstituted group of the following formula (2) 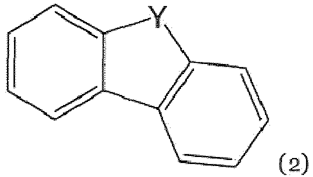 wherein Y is independently selected from the group consisting of CR'₂, SiR'₂, S and O, wherein R' is independently selected from the group consisting of C₁ to C₁₂ alkyl and C₆ to C₁₈ aryl.

R may be independently selected from the group consisting of substituted or unsubstituted C₁₀ to C₁₂ aryl and a substituted or unsubstituted group of the following formula (2) 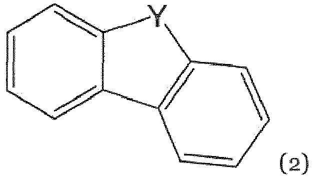 wherein Y is independently selected from the group consisting of CR'₂ and O, wherein R' is independently selected from C₁ to C₄ alkyl, especially methyl.

R may be independently selected from the following groups 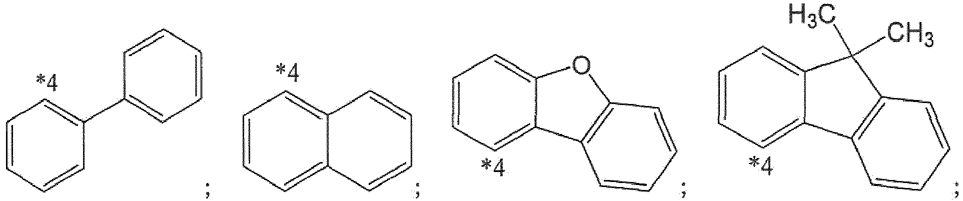 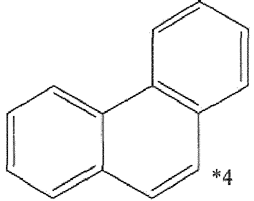 wherein R is bonded the ring Z at ^{∗}4.

R may be independently selected from the following groups 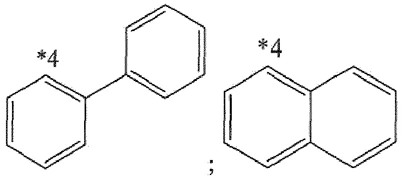 wherein R is bonded the ring Z at ^{∗}4.

X and both R may be selected the same. That is, in such a case, if X is C₆ aryl (phenylene) than both R are likewise C₆ aryl (phenyl).

The compound of formula (I) may have a LUMO energy level in the absolute scale taking vacuum energy level as zero, computed by the TURBOMOLE V6.5 program package using hybrid functional B3LYP and Gaussian 6-31G^{∗} basis set in the range from -1,90 eV to -1.70 eV. The compound of formula (I) may have a LUMO energy level in the absolute scale taking vacuum energy level as zero, computed by the TURBOMOLE V6.5 program package using hybrid functional B3LYP and Gaussian 6-31G^{∗} basis set in the range from -1,85 eV to -1.75 eV. The compound of formula (I) may have a LUMO energy level in the absolute scale taking vacuum energy level as zero, computed by the TURBOMOLE V6.5 program package using hybrid functional B3LYP and Gaussian 6-31G^{∗} basis set in the range from -1,83 eV to -1.78 eV.

The compound of formula (I) may have a refractive index at a wavelength of 633 nm in the range from 1.5 to 2.0, alternatively from 1.6 to 1.9, alternatively from 1.7 to 1.8, such as from 1.738 to 1.763.

The compound of formula (I) may have a glass transition temperature T_{g} of more than 93°C. The compound of formula (I) may have a glass transition temperature T_{g} of more than 95°C. The compound of formula (I) may have a glass transition temperature T_{g} of more than 100°C. The compound of formula (I) may have a glass transition temperature T_{g} of more than 105°C. The compound of formula (I) may have a glass transition temperature T_{g} of more than 110°C. The compound of formula (I) may have a glass transition temperature T_{g} of more than 115°C. The compound of formula (I) may have a glass transition temperature T_{g} of more than 120°C.

The compound of formula (I) may have a glass transition temperature T_{g} from 93 to 200°C. The compound of formula (I) may have a glass transition temperature T_{g} from 95 to 190°C. The compound of formula (I) may have a glass transition temperature T_{g} from 100 to 180°C. The compound of formula (I) may have a glass transition temperature T_{g} from 105 to 170°C. The compound of formula (I) may have a glass transition temperature T_{g} from 110 to 160°C. The compound of formula (I) may have a glass transition temperature T_{g} from 115 to 150°C. The compound of formula (I) may have a glass transition temperature T_{g} from 120 to 140°C.

The compound of formula (I) may comprise 8 to 13 aromatic or heteroaromatic rings, optionally 8 to 12 aromatic or heteroaromatic rings, optionally 9 to 11 aromatic or heteroaromatic rings.

The compound of formula (I) may comprise 8 to 13 aromatic or heteroaromatic rings, wherein 1 to 3 of the aromatic or heteroaromatic rings are N-containing rings; optionally 8 to 11 aromatic or heteroaromatic rings, wherein 1 to 3 of the aromatic or heteroaromatic rings are N-containing rings; optionally 8 to 12 aromatic or heteroaromatic rings, wherein 1 or 2 of the aromatic or heteroaromatic rings are N-containing rings; and optionally 9 to 11 aromatic or heteroaromatic rings wherein one of the aromatic or heteroaromatic rings is a N-containing rings.

The compound of formula (I) may comprise 8 to 13 aromatic or heteroaromatic rings, wherein 7 to 11 of the aromatic or heteroaromatic rings are aryl rings; optionally 8 to 12 aromatic or heteroaromatic rings, wherein 7 to 10 of the aromatic or heteroaromatic rings are aryl rings; optionally 9 to 11 aromatic or heteroaromatic rings, wherein 8 to 10 of the aromatic or heteroaromatic rings are aryl rings.

The compound of formula (I) may have a ratio of aryl rings to N-containing rings may be from 13:1 to 8:1, such as from 11:1 to 9:1.

The compound of formula (I) may be selected from the following compounds I-1 to I-20

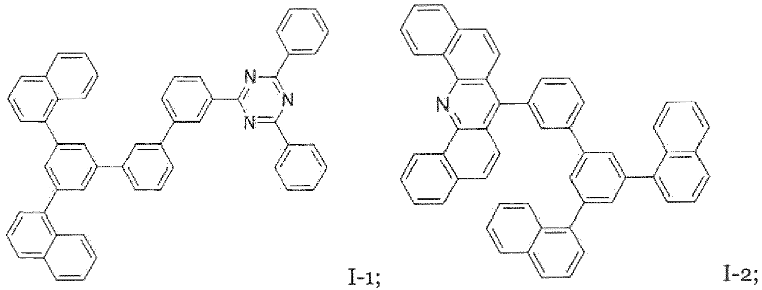

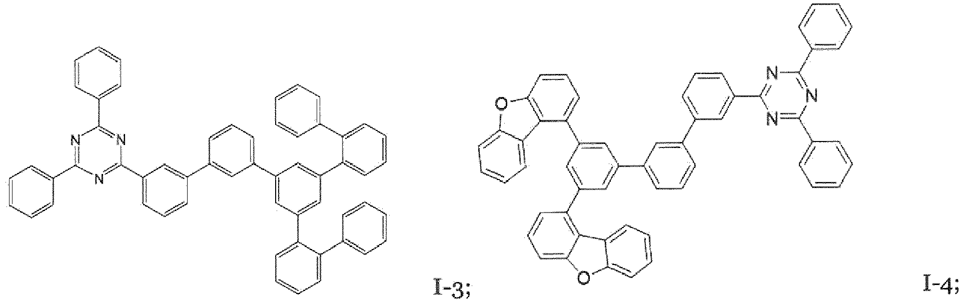

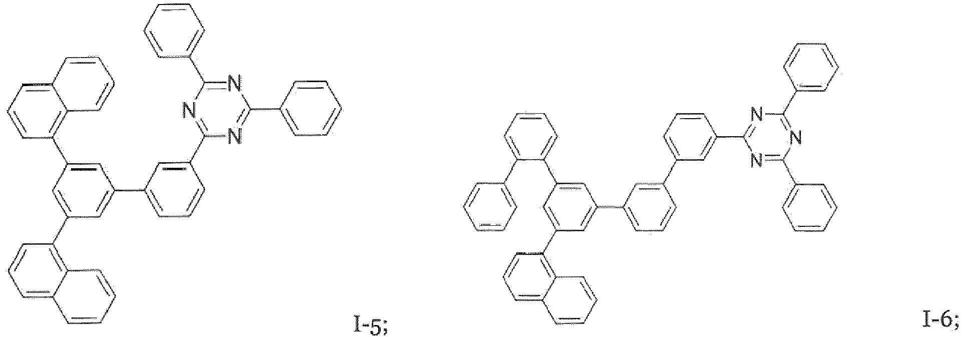

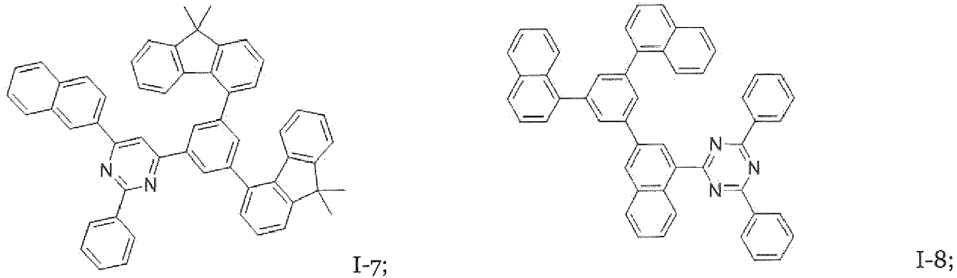

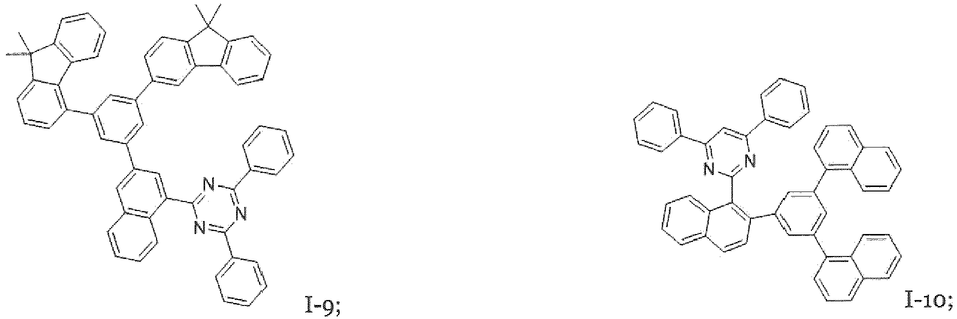

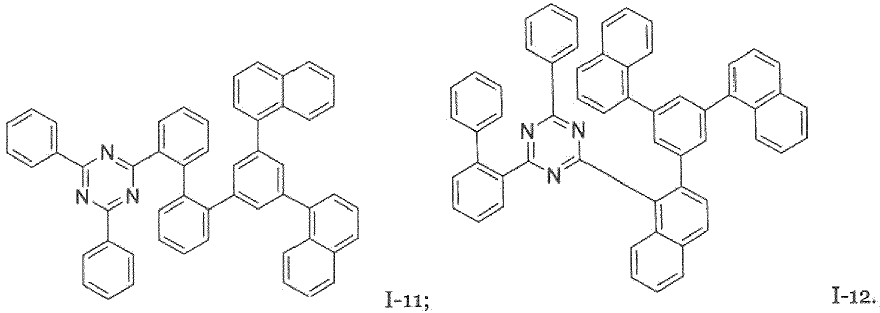

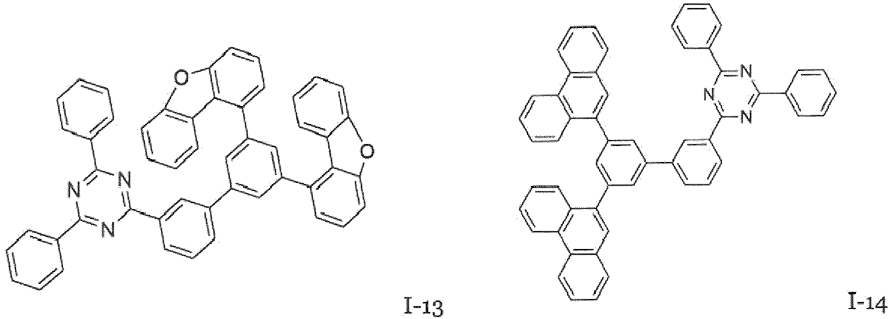

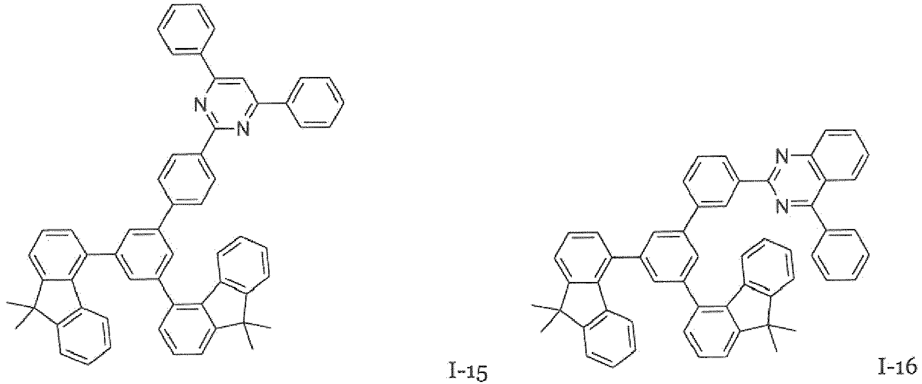

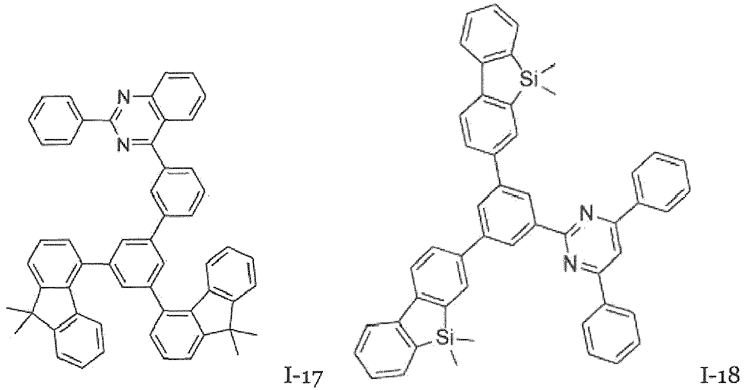

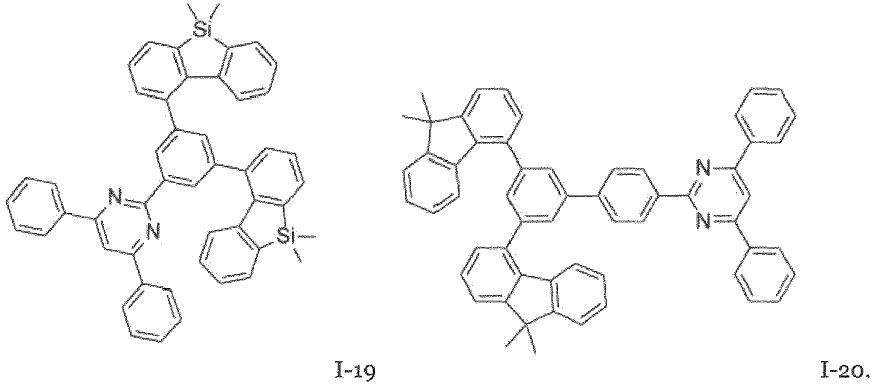

It may be provided that the compound of formula (I) does not comprise a hole transporting moiety.

It may be provided that the compound of formula (I) does not comprise a sp³ N-atom.

It may be provided that the compound of formula (I) does not comprise a triarylamine moiety.

It may be provided that the compound of formula (I) does not comprise carbazole.

### Exemplary embodiments of the compound of formula (I)

According to one embodiment, there is provided an organic light emitting diode, comprising a non-transparent substrate, an anode, a cathode, an emission layer, an electron injection layer and an electron transport layer stack;
wherein
- the electron transport layer stack is arranged between the emission layer and the electron injection layer;
- the electron transport layer stack comprises a first electron transport layer and a second electron transport layer;
- the first electron transport layer is in direct contact with the emission layer;
- the first electron transport layer comprises a compound of Formula (I) 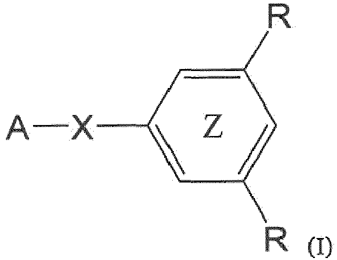 wherein
- A is an unsubstituted or substituted C₃ to C₂₁ heteroaryl comprising at least one six-membered ring, wherein the six-membered ring comprises at least two N-atoms;
   - wherein the substituted C₃ to C₂₁ heteroaryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R is independently selected from the group consisting of substituted or unsubstituted C₁₀ to C₁₈ aryl and a substituted or unsubstituted group of the following formula (2); 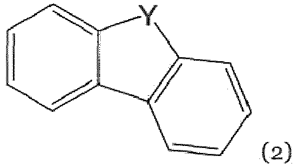
   - wherein Y is independently selected from the group consisting of CR'₂, SiR'₂, S and O, wherein R' is independently selected from C₁ to C₁₂ alkyl;
   - wherein the substituted C₁₀ to C₁₈ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
   - wherein the substituted group of the following formula (2) is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R comprises two 6-membered aryl rings;
- R is bonded to the phenylene-ring Z by a first C-atom of one of the two 6-membered aryl rings; and the first C-atom is adjacent to a second C-atom, wherein the second C-atom is a sp²-C-atom and the second C-atom is not bonded to H;
- X is a single bond or substituted or unsubstituted C₆ to C₁₈ aryl;
   - wherein the substituted C₆ to C₁₈ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- X is bonded to a C-atom of the six-membered ring comprising at least two N-atoms of A; and
- the second electron transport layer does not comprise a compound according to formula (I).

According to one embodiment, there is provided an organic light emitting diode, comprising a non-transparent substrate, an anode, a cathode, an emission layer, an electron injection layer and an electron transport layer stack;
wherein
- the electron transport layer stack is arranged between the emission layer and the electron injection layer;
- the electron transport layer stack comprises a first electron transport layer and a second electron transport layer;
- the first electron transport layer is in direct contact with the emission layer;
- the first electron transport layer comprises a compound of Formula (I) 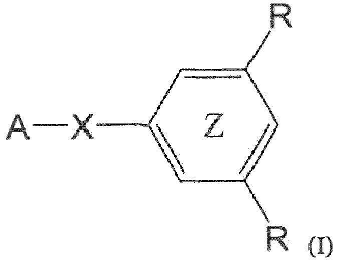 wherein
- A is an unsubstituted or substituted C₃ to C₁₅ heteroaryl comprising at least one six-membered ring, wherein the six-membered ring comprises at least two N-atoms;
   - wherein the substituted C₃ to C₂₁ heteroaryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R is independently selected from the group consisting of substituted or unsubstituted C₁₀ to C₁₂ aryl and a substituted or unsubstituted group of the following formula (2); 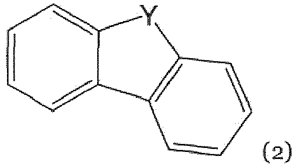
   - wherein Y is independently selected from the group consisting of CR'₂ and O, wherein R' is independently selected from C₁ to C₁ alkyl, especially methyl;
   - wherein the substituted C₁₀ to C₁₂ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
   - wherein the substituted group of the following formula (2) is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
   - R comprises two 6-membered aryl rings;
   - R is bonded to the phenylene-ring Z by a first C-atom of one of the two 6-membered aryl rings; and the first C-atom is adjacent to a second C-atom, wherein the second C-atom is a sp²-C-atom and the second C-atom is not bonded to H;
   - X is a single bond or substituted or unsubstituted C₆ to C₁₂ aryl;
      - wherein the substituted C₆ to C₁₂ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
   - X is bonded to a C-atom of the six-membered ring comprising at least two N-atoms of A; and
   - the second electron transport layer does not comprise a compound according to formula (I).

According to one embodiment, there is provided an organic light emitting diode, comprising a non-transparent substrate, an anode, a cathode, an emission layer, an electron injection layer and an electron transport layer stack;
wherein
- the electron transport layer stack is arranged between the emission layer and the electron injection layer;
- the electron transport layer stack comprises a first electron transport layer and a second electron transport layer;
- the first electron transport layer is in direct contact with the emission layer;
- the first electron transport layer comprises a compound of Formula (I) 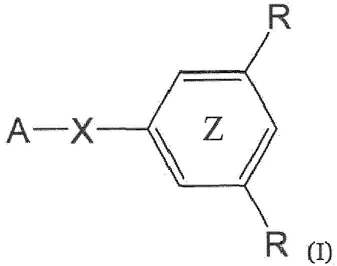 wherein
- A has one of the following formulas 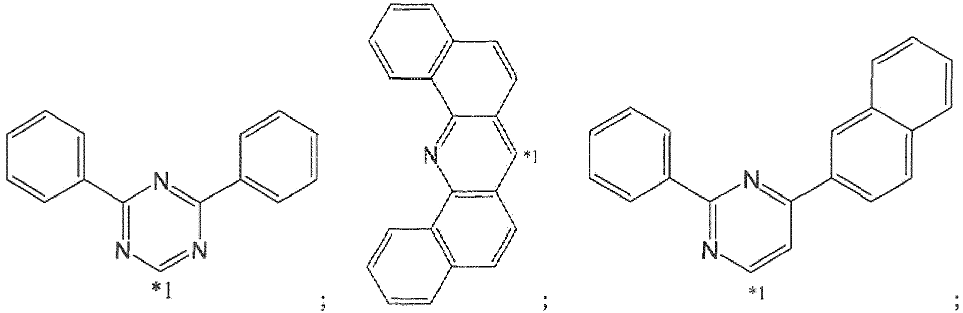 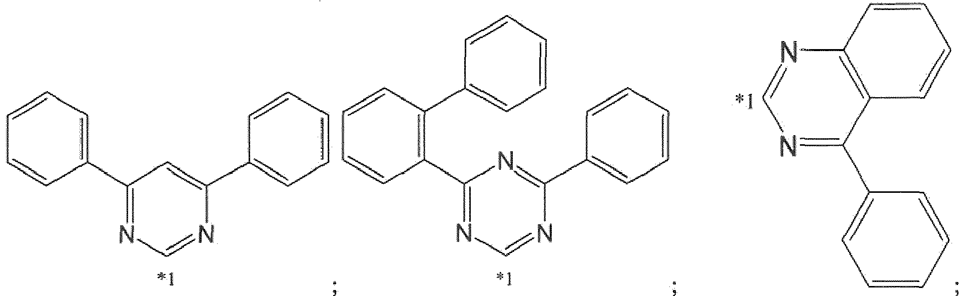 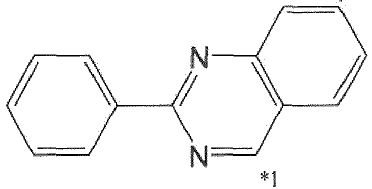 X is selected from a single bond or from the following formulas 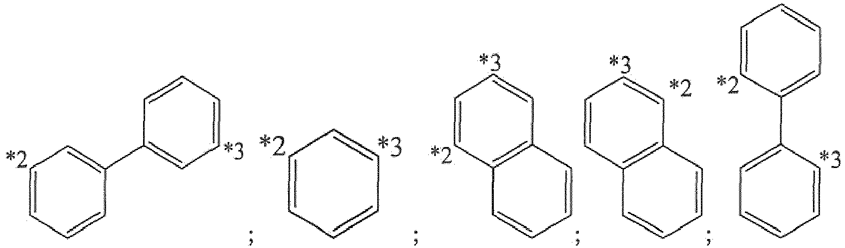
- R is independently selected from the following groups 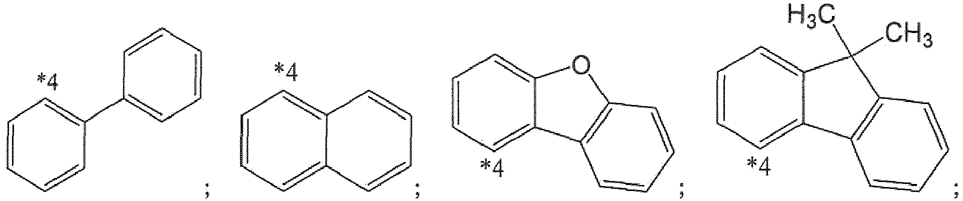 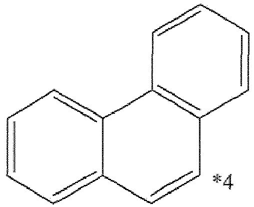 wherein R is bonded the ring Z at ^{∗}4
- the second electron transport layer does not comprise a compound according to formula (I).

### First electron transport layer

The first electron transport layer may comprise more than one compound of formula (I). The first electron transport layer may consist of a mixture of two or more compounds of formula (I). The first electron transport layer may consist of one compound of formula (I).

The first electron transport layer may be free of an electrical dopant, such as an n-type dopant, especially a redox n-type dopant.

The electron transport layer is free of an electrical dopant, such as an n-type dopant, especially a redox n-type dopant. The term "free of" in this regard does not exclude impurities. Impurities have no technical effect with respect to the object achieved by the present invention. Impurities are not deliberately added to the layer during processing.

The term "free of" a compound means that such compound is not deliberately added to the layer during processing.

Under electrical dopant, especially n-type dopant it is understood a compound which, if embedded into an electron transport matrix, improves, in comparison with the neat matrix under the same physical conditions, the electron properties of the formed organic material, particularly in terms of electron injection and/or electron conductivity.

In the context of the present invention "embedded into an electron transport matrix" means homogenously mixed with the electron transport matrix.

The electrical dopant as referred to herein is especially selected from elemental metals, metal salts, metal complexes and organic radicals.

In one embodiment, the electrical dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,
- the lithium complex has the formula II, III or IV: 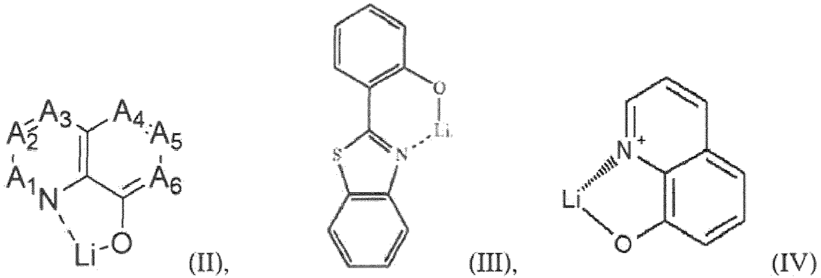 wherein
   A₁ to A₆ are same or independently selected from CH, CR, N, O;
   R is same or independently selected from hydrogen, halogen, alkyl or aryl or heteroaryl with 1 to 20 carbon atoms; and more preferred A₁ to A₆ are CH,
- the borate based organic ligand is a tetra(1H-pyrazol-1-yl)borate,
- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
- the pyridinolate is a 2-(diphenylphosphoryl)pyridin-3-olate,

- the lithium Schiff base has the structure 100, 101, 102 or 103:

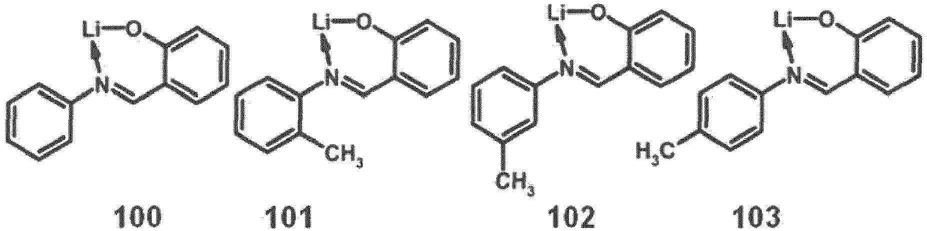

According to one embodiment of the invention, the electron transport layer of the present invention is free of a lithium organic complex, alternatively 8-Hydroxyquinolinolato-lithium (= LiQ).

According to one embodiment of the present invention the electron transport layer is free of a metal, preferably selected from alkali metals, alkaline earth metals, rare earth metals and metals of the first transition period Ti, V, Cr and Mn, especially selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The most practical benchmark for the strength of an n-dopant is the value of its redox potential. There is no particular limitation in terms how negative the value of the redox potential can be.

The measurement of redox potentials is practically performed for a corresponding redox couple consisting of the reduced and of the oxidized form of the same compound.

In case that the n-type dopant is an electrically neutral metal complex and/or an electrically neutral organic radical, the measurement of its redox potential is actually performed for the redox couple formed by
(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

Preferably, the redox potential of the electrically neutral metal complex and/or of the electrically neutral organic radical may have a value which is more negative than - 0.5 V, preferably more negative than - 1.2 V, more preferably more negative than - 1.7 V, even more preferably more negative than - 2.1 V, most preferably more negative than - 2.5 V, if measured by cyclic voltammetry against ferrocene/ferrocenium reference redox couple for a corresponding redox couple consisting of
( i) the electrically neutral metal complex and its cation radical formed by an abstraction of one electron from the electrically neutral metal complex, or
( ii) the electrically neutral organic radical and its cation formed by an abstraction of one electron from the electrically neutral organic radical.

In a preferred embodiment, the redox potential of the n-dopant is between the value which is about 0.5 V more positive and the value which is about 0.5 V more negative than the value of the reduction potential of the chosen electron transport matrix.

Electrically neutral metal complexes suitable as n-type dopants may be e.g. strongly reductive complexes of some transition metals in low oxidation state. Particularly strong n-type dopants may be selected for example from Cr(II), Mo(II) and/or W(II) guanidinate complexes such as W2(hpp)4, as described in more detail in WO2005/086251.

Electrically neutral organic radicals suitable as n-type dopants may be e.g. organic radicals created by supply of additional energy from their stable dimers, oligomers or polymers, as described in more detail in EP 1 837 926 B1, WO2007/107306, or WO2007/107356. Under an elemental metal, it is understood a metal in a state of a neat metal, of a metal alloy, or in a state of free atoms or metal clusters. It is understood that metals deposited by vacuum thermal evaporation from a metallic phase, e.g. from a neat bulk metal, vaporize in their elemental form. It is further understood that if the vaporized elemental metal is deposited together with a covalent matrix, the metal atoms and/or clusters are embedded in the covalent matrix. In other words, it is understood that any metal doped covalent material prepared by vacuum thermal evaporation contains the metal at least partially in its elemental form.

For the use in consumer electronics, only metals containing stable nuclides or nuclides having very long halftime of radioactive decay might be applicable. As an acceptable level of nuclear stability, the nuclear stability of natural potassium can be taken.

In one embodiment, the electrical may be selected from electropositive metals selected from alkali metals, alkaline earth metals, rare earth metals and metals of the first transition period Ti, V, Cr and Mn. Preferably, the n-dopant may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

### Second electron transport layer

The organic light emitting device comprises a second electron transport layer (ETL). By suitably adjusting energy levels of particular layers of the ETL, the injection and transport of the electrons may be controlled, and the holes may be efficiently blocked. Thus, the OLED may have long lifetime.

The electron transport layer of the semiconducting device may comprise the first organic compound as defined above as the organic electron transport matrix (ETM) material. The electron transport layer may comprise, besides or instead of the first organic compound, further ETM materials known in the art. Likewise, the electron transport layer may comprise as the only electron transport matrix material the first organic compound. In case that the inventive organic semiconducting device comprises more than one electron transport layers, the first organic compound may be comprised in only one of the electron transport layers, in more than one of the electron transport layers or in all of the electron transport layers. In accordance with the invention, the electron transport layer may comprise, besides the ETM material, at least one additive as defined below.

Further, the electron transport layer may comprise one or more additives. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another emdodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound represented by the general Formula (1) as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP1 970 371 A1 or WO 2013/079217 A1.

According to an embodiment, the second electron transport layer comprises an electron transport compound (which is not a compound of formula (I)), wherein the electron transport compound comprises 8 to 13 aromatic or heteroaromatic rings, optionally 8 to 11 aromatic or heteroaromatic rings, optionally 9 to 11 aromatic or heteroaromatic rings, and optionally 9 aromatic or heteroaromatic rings, wherein one or more of the aromatic or heteroaromatic rings may be substituted with C1 to C4 alkyl. In this regard, an aromatic, respectively heteroaromatic ring is a single aromatic ring, for example a 6-membered aromatic ring such as phenyl, a 6-membered heteroaromatic ring such as pyridyl, a 5-membered heteroaromatic ring such as pyrrolyl etc. In a system of condensed (hetero)aromatic rings, each ring is considered as a single ring in this regard. For example, naphthalene comprises two aromatic rings.

The electron transport compound may comprise at least one heteroaromatic ring, optionally 1 to 5 heteroaromatic rings, optionally 1 to 4 heteroaromatic rings, optionally 1 to 3 heteroaromatic rings, and optionally 1 or 2 heteroaromatic rings.

The aromatic or heteroaromatic rings of the electron transport compound may be 6-membered rings.

The heteroaromatic rings of the electron transport compound may be a N-containing heteroaromatic ring, optionally all of the heteroaromatic rings are N-containing heteroaromatic rings, optionally all of the heteroaromatic rings heteroaromatic rings contain N as the only type of heteroatom.

The electron transport compound may comprise at least one six-member heteroaromatic ring containing one to three N-atoms in each heteroaromatic ring, optionally one to three 6-membered heteroaromatic rings containing one to three N-atoms in each heteroaromatic ring, respectively.

The at least one 6-membered heteroaromatic ring comprised in the electron transport compound may be an azine. The at least one 6-membered heteroaromatic ring comprised in the electron transport compound may be triazine, diazine, pyrazine, pyrimidine, pyridine, quinazoline or bonzoquinazoline, preferably triazine.

If the electron transport compound comprises two or more heteroaromatic rings, the heteroaromatic rings may be separated from each other by at least one aromatic ring which is free of a heteroatom.

In an embodiment, the heteroatoms in the heteroaromatic rings of the electron transport compound are bound into the molecular structure of the electron transport compound by at least one double bond.

According to an embodiment, wherein the compound comprising at least one nitrogen atom in a six-member aromatic ring in the electron transport layer is selected from formula (xxa) or formula (xxb):

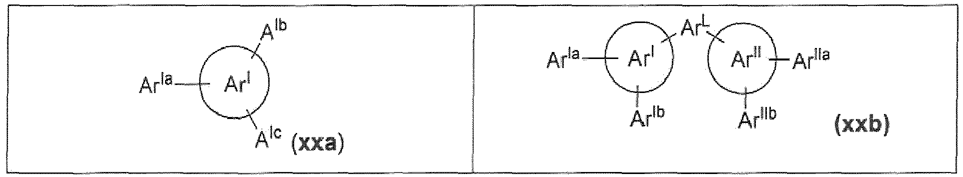

wherein Ar^{I} is a substituted or unsubstituted C₃ to C₄₀ heteroaromatic ring system comprising at least one nitrogen atom,
wherein Ar^{II} is substituted or unsubstituted C₃ to C₄₀ heteroaromatic ring system comprising at least one nitrogen atom,
wherein the substituents on Ar^{I} and Ar^{II} are, identically or differently on each occurrence, D, a monovalent aromatic or heteroaromatic ring system having 5 to 60 aromatic ring atoms, which may in each case be substituted by one or more radicals R^{a};
wherein Ar^{Ia}, Ar^{Ib}, Ar^{Ic}, Ar^{IIa}, and Ar^{IIb} are, identically or differently on each occurrence, H, D, a monovalent aromatic or heteroaromatic ring system having 5 to 60 aromatic ring atoms, which may in each case be substituted by one or more radicals R^{a};
wherein at least one of Ar^{Ia}, Ar^{Ib}, Ar^{Ic} in formula (xxa) and in case of formula (II) at least one of
Ar^{Ia}, Ar^{Ib}, Ar^{IIa}, and Ar^{IIb} is independently selected from a monovalent aromatic or heteroaromatic ring system having 5 to 60 aromatic ring atoms, which may in each case be substituted by one or more radicals R^{a};
wherein Ar^{L} is a divalent aromatic or heteroaromatic ring system having 5 to 60 aromatic ring atoms, which is optionally substituted by one or more radicals R^{a};
wherein R^{a} is on each occurrence, identically or differently, H, D, F, Cl, Br, I, CHO, N(R^{b})₂, N(Ar^{1s})₂, B(Ar^{1s})₂, C(=O)Ar^{1s}, P(=Y)(R^{c})₂, S(=O)Ar^{s1}, S(=O)₂Ar^{1s}, CR^{b}=CR^{b}Ar^{1s}, CN, NO₂, Si(R^{b})₃, B(OR^{b})₂, B(R^{b})₂, B(N(R^{b})₂)₂, OSO₂R^{b}, a substituted or unsubstituted straight-chain C₁ to C₂₀ alkyl, a substituted or unsubstitutedstraight-chain C₁ to C₂₀ alkenyl, a substituted or unsubstituted straight-chain C₁ to C₂₀ alkynyl, a substituted or unsubstituted straight-chain C₁ to C₂₀ alkoxy, a substituted or unsubstituted straight-chain C₁ to C₂₀ thioalkoxy, substituted or unsubstituted branched C₃ to C₂₀ alkyl, a substituted or unsubstituted branched C₃ to C₂₀ alkenyl, a substituted or unsubstituted branched C₃ to C₂₀ a substituted or unsubstituted branched C₃ to C₂₀ alkynyl, a substituted or unsubstituted branched C₃ to C₂₀ alkoxy or a substituted or unsubstituted C₃ to C₂₀ branched thioalkoxy, substituted or unsubstituted cyclic C₃ to C₄, alkyl, substituted or unsubstituted cyclic C₃ to C₄₀ alkenyl, substituted or unsubstituted cyclic C₃ to C₄₀ alkinyl, substituted or unsubstituted cyclic C₃ to C₄₀ alkoxy or substituted or unsubstituted cyclic C₃ to C₄₀ thioalkoxy; substituted or unsubstituted heterocyclic C₃ to C₄₀ alkyl, substituted or unsubstituted heterocyclic C₃ to C₄₀ alkenyl, substituted or unsubstituted heterocyclic C₃ to C₄₀ alkynyl, substituted or unsubstituted heterocyclic C₃ to C₄₀ alkoxy or substituted or unsubstituted heterocyclic C₃ to C₄₀ thioalkoxy; wherein the one or more substituents, if present, is selected from R^{b}, where one or more non-adjacent CH2 groups is optionally replaced by R^{b}C=CR^{b}, C≡C, Si(R^{b})₂, Ge(R^{b})₂, Sn(R^{b})₂, C=O, C=S, C=Se, C=NR^{b}, P(=O)(R^{b}), SO, SO₂, NR^{b}, O, S or CONR^{b} and where one or more H atoms is optionally replaced by D, F, Cl, Br, I, CN or NO₂, or an aromatic or heteroaromatic ring system having 5 to 60 aromatic ring atoms, which may in each case be substituted by one or more radicals R^{b}, or an aryloxy or heteroaryloxy group having 5 to 60 aromatic ring atoms, which is optionally substituted by one or more radicals R^{b}, or a combination of these systems; two or more adjacent substituents R^{a} here optionally forms a mono- or polycyclic, aliphatic or aromatic ring system with one another;
wherein Ar^{1s} is on each occurrence, identically or differently, an aromatic or heteroaromatic ring system having 5 to 30 aromatic ring atoms, which is optionally substituted by one or more radicals R^{b}; two radicals Ar^{1s} here which are bonded to the same nitrogen, phosphorus or boron atom may also be linked to one another by a single bond or a bridge selected from B(R^{b}), C(R^{b})₂, Si(R^{b})₂, C=O, C=NR^{b}, C=C(R^{b})₂, O, S, S=O, SO₂, N(R^{b}), P(R^{b}) and P(=Y)R^{c};
wherein R^{b} is on each occurrence, identically or differently, H, D or a C₁ to C₂₀ aliphatic hydrocarbyl, a C₁ to C₂₀ aryl and/or C₁ to C₂₀ heteroaryl, in which, in addition, H atoms is optionally replaced by D or F; two or more adjacent substituents R^{b} here may also form a mono- or polycyclic, aliphatic or aromatic ring system with one another;
wherein Y is selected from O, S or Se, preferably O, and R^{c} is independently selected from C₆ to C₁₂ aryl, C₃ to C₁₂ heteroaryl, C₁ to C₆ alkyl, C₁ to C₆ alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy;

For the purposes of this invention, an aromatic or heteroaromatic ring system is intended to be taken to mean a system which does not necessarily contain only one aryl or one heteroaryl group or only aryl or heteroaryl groups, but instead in which a plurality of aryl or heteroaryl groups may also be interrupted by a short non-aromatic unit (preferably less than 10 percent of the atoms other than H), such as, for example, an sp³-hybridised C, N or O atom. Thus, for example, systems such as 9,9'-spirobifluorene, 9,9-diarylfluorene, triarylamine, diaryl ether, stilbene, benzophenone, etc., are also intended to be taken to mean aromatic ring systems for the purposes of this invention. Likewise, an aromatic or heteroaromatic ring system is taken to mean systems in which a plurality of aryl or heteroaryl groups are linked to one another by single bonds, for example biphenyl, terphenyl or bipyridine.

According to one embodiment, Ar^{I} is selected from pyrazine, pyridine, pyrimidine, quinazoline, benzoquinazoline or triazine, phenanthroline.

According to one embodiment, Ar^{II} is selected from pyrazine, pyridine, pyrimidine, quinazoline, benzoquinazoline, or triazine, phenanthroline.

According to one embodiment, Ar^{I} in formula (xxa) or (xxxa) is selected from pyrazine, pyridine, pyrimidine, triazine, phenanthroline; quinazoline, benzoquinazoline or Ar^{I} in formula (xxa) or (xxxa) and Ar^{II} in formula (xxb) or (xxxb) are independently of each other selected from pyrazine, pyridine, pyrimidine, triazine, or phenanthroline, quinazoline, benzoquinazoline.

According to an embodiment, wherein the compound comprising at least one nitrogen atom in a six-member aromatic ring in the electron transport layer is selected from formula (xxxa) or formula (xxxb): 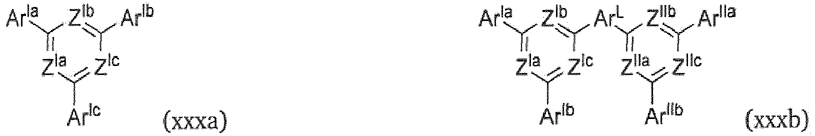 wherein
Z^{Ia} is selected from N or CH,
Z^{Ib} is selected from N or CH,
Z^{Ic} is selected from N or CH,
Z^{IIa} is selected from N or CH,
Z^{IIb} is selected from N or CH, and
Z^{IIc} is selected from N or CH,
wherein in formula (XXxa) at least one of Z^{Ia}, Z^{Ib} and Z^{Ic} is selected from N,
wherein in formula (XXxb) at least one of Z^{Ia}, Z^{Ib}, Z^{Ic}, Z^{IIa}, Z^{IIb}, and Z^{IIc} is selected from N,
wherein Ar^{Ia}, Ar^{Ib}, Ar^{Ic}, Ar^{IIa} and Ar^{IIb} are identically or differently on each occurrence, a monovalent aromatic or heteroaromatic ring system having 5 to 60 aromatic ring atoms, which may in each case be substituted by one or more radicals R^{a};
wherein Ar^{L} is a divalent aromatic or heteroaromatic ring system having 5 to 60 aromatic ring atoms, which is optionally substituted by one or more radicals R^{a},
wherein R^{a} is on each occurrence, identically or differently, H, D, F, Cl, Br, I, CHO, N(R^{b})₂, N(Ar^{1s})₂, B(Ar^{1s})₂, C(=O)Ar^{1s}, P(=Y)(R^{c})₂, S(=O)Ar^{1s}, S(=O)₂Ar^{1s}, CR^{b}=CR^{b}Ar^{1s}, CN, NO₂, Si(R^{b})₃, B(OR^{b})₂, B(R^{b})₂, B(N(R^{b})₂)₂, OSO₂R^{b}, a substituted or unsubstituted straight-chain C₁ to C₂₀ alkyl, a substituted or unsubstitutedstraight-chain C₁ to C₂₀ alkenyl, a substituted or unsubstituted straight-chain C₁ to C₂₀ alkynyl, a substituted or unsubstituted straight-chain C₁ to C₂₀ alkoxy, a substituted or unsubstituted straight-chain C₁ to C₂₀ thioalkoxy, substituted or unsubstituted branched C₃ to C₂₀ alkyl, a substituted or unsubstituted branched C₃ to C₂₀ alkenyl, a substituted or unsubstituted branched C₃ to C₂₀ a substituted or unsubstituted branched C₃ to C₂₀ alkynyl, a substituted or unsubstituted branched C₃ to C₂₀ alkoxy or a substituted or unsubstituted branched C₃ to C₂₀ thioalkoxy,substituted or unsubstituted cyclic C₃ to C₄₀ alkyl, substituted or unsubstituted cyclic C₃ to C₄₀ alkenyl, substituted or unsubstituted cyclic C₃ to C₄₀ alkinyl, substituted or unsubstituted cyclic C₃ to C₄₀ alkoxy or substituted or unsubstituted cyclic C₃ to C₄₀ thioalkoxy;substituted or unsubstituted heterocyclic C₃ to C₄₀ alkyl, substituted or unsubstituted heterocyclic C₃ to C₄₀ alkenyl, substituted or unsubstituted heterocyclic C₃ to C₄₀ alkynyl, substituted or unsubstituted heterocyclic C₃ to C₄₀ alkoxy or substituted or unsubstituted heterocyclic C₃ to C₄₀ thioalkoxy;
wherein the one or more substituents, if present, is selected from R^{b}, where one or more non-adjacent CH₂ groups is optionally replaced by R^{b}C=CR^{b}, O≡C, Si(R^{b})₂, Ge(R^{b})₂, Sn(R^{b})₂, C=O, C=S, C=Se, C=NR^{b}, P(=O)(R^{b}), SO, SO₂, NR^{b}, O, S or CONR^{b} and where one or more H atoms is optionally replaced by D, F, Cl, Br, I, CN or NO₂, or an aromatic or heteroaromatic ring system having 5 to 60 aromatic ring atoms, which may in each case be substituted by one or more radicals R^{b}, or an aryloxy or heteroaryloxy group having 5 to 60 aromatic ring atoms, which is optionally substituted by one or more radicals R^{b}, or a combination of these systems; two or more adjacent substituents R^{a} here optionally forms a mono- or polycyclic, aliphatic or aromatic ring system with one another;
wherein Ar^{1s} is on each occurrence, identically or differently, an aromatic or heteroaromatic ring system having 5 to 30 aromatic ring atoms, which is optionally substituted by one or more radicals R^{b}; two radicals Ar^{1s} here which are bonded to the same nitrogen, phosphorus or boron atom may also be linked to one another by a single bond or a bridge selected from B(R^{b}), C(R^{b})₂, Si(R^{b})₂, C=O, C=NR^{b}, C=C(R^{b})₂, O, S, S=O, SO₂, N(R^{b}), P(R^{b}) and P(=Y)R^{c};
wherein R^{b} is on each occurrence, identically or differently, H, D or a C₁ to C₂₀ aliphatic hydrocarbyl, a C₁ to C₂₀ aryl and/or C₁ to C₂₀ heteroaryl, in which, in addition, H atoms is optionally replaced by D or F; two or more adjacent substituents R^{b} here may also form a mono- or polycyclic, aliphatic or aromatic ring system with one another;
wherein Y is selected from O, S or Se, preferably O, and R^{c} is independently selected from C₆ to C₁₂ aryl, C₃ to C₁₂ heteroaryl, C₁ to C₆ alkyl, C₁ to C₆ alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy.

For the purposes of this invention, an aromatic or heteroaromatic ring system is intended to be taken to mean a system which does not necessarily contain only aryl or heteroaryl groups, but instead in which a plurality of aryl or heteroaryl groups may also be interrupted by a short non-aromatic unit (preferably less than 10 percent of the atoms other than H), such as, for example, an sp³-hybridised C, N or O atom. Thus, for example, systems such as 9,9'-spirobifluorene, 9,9-diarylfluorene, triarylamine, diaryl ether, stilbene, benzophenone, etc., are also intended to be taken to mean aromatic ring systems for the purposes of this invention. Likewise, an aromatic or heteroaromatic ring system is taken to mean systems in which a plurality of aryl or heteroaryl groups are linked to one another by single bonds, for example biphenyl, terphenyl or bipyridine.

### Organic light emitting diode

The first electron layer may also be referred to as auxiliary electron transport layer (a-ETL) or as hole blocking layer (HBL).

The first electron transport layer may be in direct contact with the second electron transport layer.

In accordance with the invention, the organic light emitting diode may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### Anode electrode

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO₂), aluminum zinc oxide (AlZO) and zinc oxide (ZnO), may be used to form the anode electrode. The anode electrode may also be formed using metals, typically silver (Ag), gold (Au), or metal alloys.

### Hole injection layer

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-i-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

### Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (1).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracence (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) which is the first electron transport layer is formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The hole blocking layer may be the inventive organic semiconductor layer comprising or consisting of the inventive compound represented by the general Formula (1) as defined above.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving

According to an embodiment, the hole blocking layer may be arranged directly sandwiched between the at least one emission layer and the second electron transport layer.

### Electron transport layer (ETL)

The OLED according to the present invention comprises the first and the second electron transport layer (ETL) and may comprise, in addition, further electron transport layers.

By suitably adjusting energy levels of particular layers of the ETL, the injection and transport of the electrons may be controlled, and the holes may be efficiently blocked. Thus, the OLED may have long lifetime.

The electron transport layer of the semiconducting device may comprise the first organic compound as defined above as the organic electron transport matrix (ETM) material. The electron transport layer may comprise, besides or instead of the first organic compound, further ETM materials known in the art. Likewise, the electron transport layer may comprise as the only electron transport matrix material the first organic compound. In case that the inventive organic semiconducting device comprises more than one electron transport layers, the first organic compound may be comprised in only one of the electron transport layers, in more than one of the electron transport layers or in all of the electron transport layers. In accordance with the invention, the electron transport layer may comprise, besides the ETM material, at least one additive as defined below.

Further, the electron transport layer may comprise one or more additives. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another emdodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound represented by the general Formula (1) as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

### Electron injection layer (EIL)

An optional EIL, which may facilitate injection of electrons from the cathode, may be formed on the ETL, preferably directly on the electron transport layer. Examples of materials for forming the EIL include lithium 8-hydroxyquinolinolate (LiQ), LiF, NaCl, CsF, Li₂O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Charge generation layer (CGL)

The charge generation layer (CGL) may comprise a p- type charge generation layer (p-CGL) and an n-type charge generation layer (n-CGL). An interlayer may be arranged between the p-CGL and the -n-CGL.

Typically, the charge generation layer is a pn junction joining an n-type charge generation layer (electron generating layer) and a hole generating layer. The n-side of the pn junction generates electrons and injects them into the layer which is adjacent in the direction to the anode. Analogously, the p-side of the p-n junction generates holes and injects them into the layer which is adjacent in the direction to the cathode.

Charge generating layers are used in tandem and stacked devices, for example, in tandem or stacked OLEDs comprising, between two electrodes, two or more emission layers. In a tandem or stacked OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, FeCl₃, FeF₃, and SbCl₅. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generating layer may be the layer comprising the compound of Formula (I). The n-type charge generation layer can be layer of a neat n-type dopant, for example of a metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Li, Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline)aluminum, benzazole derivatives, and silole derivatives.

The hole generating layer may be arranged in direct contact to the n-type charge generation layer.

According to one aspect of the present invention, there is provided an organic light-emitting diode (OLED) comprising: a substrate; an anode electrode formed on the substrate; a hole injection layer, a hole transport layer, an emission layer, an electron transport layer stack comprising the first electron transport layer comprising the compound of formula (I) and the second electron transport layer and a cathode electrode.
a substrate; an anode electrode formed on the substrate; a hole injection layer, a hole transport layer, an electron blocking layer, an emission layer, an electron transport layer stack comprising the first electron transport layer comprising the compound of formula (I) and the second electron transport layer and a cathode electrode.

According to another aspect of the present invention, there is provided an OLED comprising: a substrate; an anode electrode formed on the substrate; a hole injection layer, a hole transport layer, an electron blocking layer, an emission layer, an electron transport layer stack comprising the first electron transport layer comprising the compound of formula (I) and the second electron transport layer, an electron injection layer, and a cathode electrode.

According to various embodiments of the present invention, there may be provided OLEDs layers arranged between the above mentioned layers, on the substrate or on the top electrode.

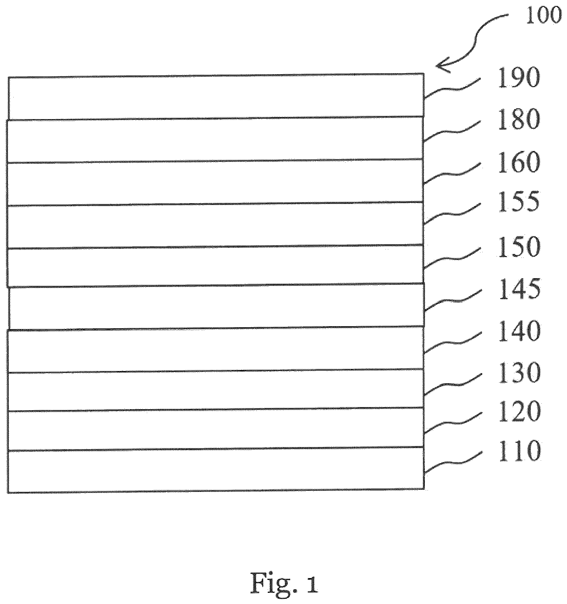

For example, the OLED according to Fig. 1 may be formed by a process, wherein on a substrate (110), an anode (120), a hole injection layer (130), a hole transport layer (140), an electron blocking layer (145), an emission layer (150), the first electron transport layer (155), the second electron transport layer (160), an electron injection layer (180) and the cathode electrode (190) are subsequently formed in that order.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device or a lighting device, most preferably a display device.

In one embodiment, the organic light emitting diode according to the invention further comprises a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb): 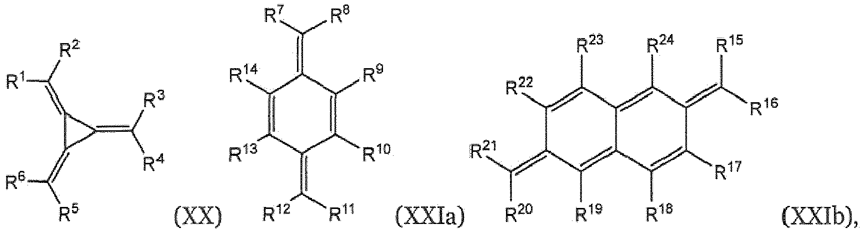 wherein (as an exception different to the description above) R¹, R², R³, R⁴, R⁵, R⁶, R⁷, R⁸, R¹¹, R¹², R¹⁵, R¹⁶, R²⁰, R²¹ are independently selected from above mentioned electron withdrawing groups and R⁹, R¹⁰, R¹³, R¹⁴, R¹⁷, R¹⁸, R¹⁹, R²², R²³ and R²⁴ are independently selected from H, halogen and above mentioned electron withdrawing groups.

### Method for manufacturing the organic electronic device

According to another aspect of the present invention, there is provided a method of manufacturing an organic light emitting diode, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spin-coating, printing, casting; and/or
- slot-die coating.

According to various embodiments of the present invention, there is provided a method using:
- a first deposition source to release the compound of Formula (I) according to the invention, and
- a second deposition source to release a compound for forming the second electron transport layer
the method comprising the steps of forming the organic semiconductor layer; whereby for an organic light-emitting diode (OLED):
- The first electron transport layer is formed by releasing the compound of formula (I) according to the invention from the first deposition source and the second electron transport layer is formed by releasing the compound of formula (II) from the second deposition source.

According to various embodiments of the present invention, the method may further include forming on the anode electrode, an emission layer and at least one layer selected from the group consisting of forming a hole injection layer, forming a hole transport layer, between the anode electrode and the first electron transport layer.

According to various embodiments of the present invention, the method may further include the steps for forming an organic light-emitting diode (OLED), wherein
- on a substrate a first anode electrode is formed,
- on the first anode electrode an emission layer is formed,
- on the emission layer an electron transport layer stack is formed, optionally a hole blocking layer is formed on the emission layer and an organic semiconductor layer is formed,
- and finally a cathode electrode is formed,
- optional a hole injection layer, a hole transport layer, and an electron blocking layer, formed in that order between the first anode electrode and the emission layer,
- the first electron transport layer and the second electron transport layer are formed between the emission layer and the cathode electrode;
- optional an electron injection layer is formed between the second electron transport layer and the cathode electrode.

According to various embodiments of the present invention, the method may further comprise forming an electron injection layer on the second electron transport layer. However, according to various embodiments of the OLED of the present invention, the OLED may not comprise an electron injection layer.

According to various embodiments, the OLED may have the following layer structure, wherein the layers having the following order:
Anode, hole injection layer, first hole transport layer, second hole transport layer, emission layer, first electron transport layer, second electron transport layer, and cathode.

### Compound of formula (II)

The first electron transport layer comprises a compound of formula (II)

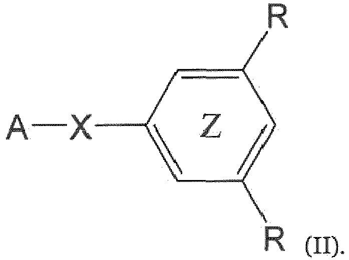

In the formula (II), the "Z" in the phenylene ring has no chemical meaning but merely serves to facilitate reference to the ring in the present disclosure. Whenever reference to the "ring Z" is made herein, reference is made to the phenylene ring connecting A-X- and the two R.

A is an unsubstituted or substituted C₃ to C₂₁ heteroaryl comprising at least one six-membered ring, wherein the six-membered ring comprises at least two N-atoms. A may be an unsubstituted or substituted C₃ to C₂₁ heteroaryl comprising at least one six-membered ring, wherein the six-membered ring comprises three N-atoms. A may be an unsubstituted or substituted C₃ to C₂₁ heteroaryl comprising at least one six-membered ring, wherein the six-membered ring is a triazine ring.

A may be an unsubstituted or substituted C₃ to C₁₅ heteroaryl comprising at least one six-membered ring, wherein the six-membered ring comprises at least two N-atoms. A may be an unsubstituted or substituted C₃ to C₁₅ heteroaryl comprising at least one six-membered ring, wherein the six-membered ring comprises three N-atoms. A may be an unsubstituted or substituted C₃ to C₁₅ heteroaryl comprising at least one six-membered ring, wherein the six-membered ring is a triazine ring.

A may have one of the following formulas 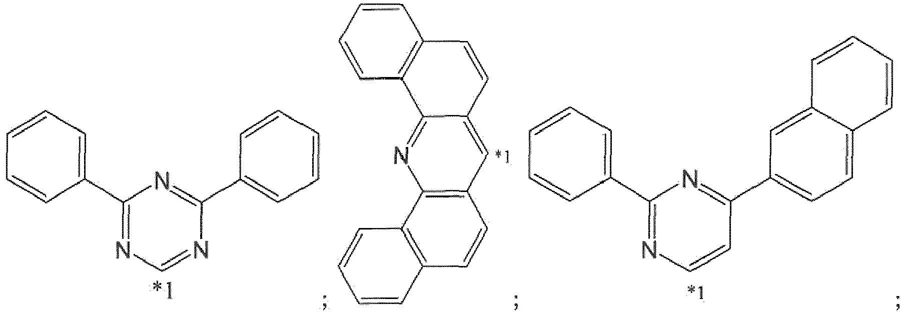 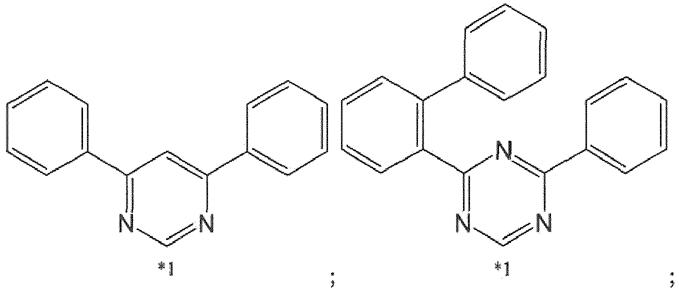 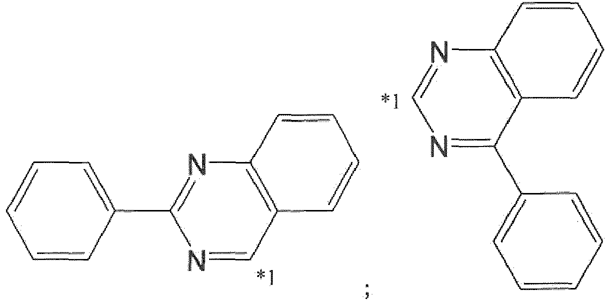 wherein A is bonded at ^{∗}1 to X.

A may have the following formula (Ia) 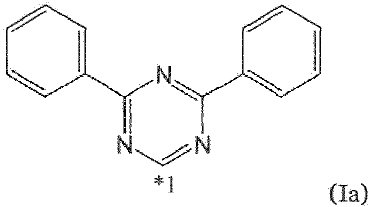 wherein Ia is bonded at *1 to X.

X is bonded to a C-atom of the six-membered ring comprising at least one N-atom of A. For example, if A has the following formula 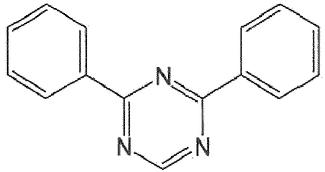 then binding to X is necessarily at the last remaining C-atom of the N-containing ring to form the following structure

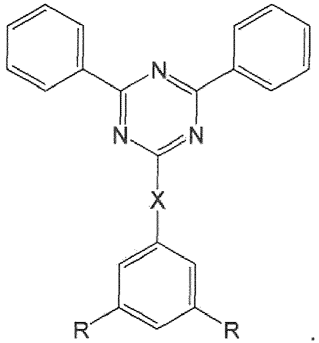

X is a single bond or substituted or unsubstituted C₆ to C₆₀ aryl. X may be a single bond or substituted or unsubstituted C₆ to C₅₄ aryl. X may be a single bond or substituted or unsubstituted C₆ to C₄₈ aryl. X may be a single bond or substituted or unsubstituted C₆ to C₄₂ aryl. X may be a single bond or substituted or unsubstituted C₆ to C₃₆ aryl. X may be a single bond or substituted or unsubstituted C₆ to C₃₀ aryl. X may be a single bond or substituted or unsubstituted C₆ to C₂₄ aryl. X may be a single bond or substituted or unsubstituted C₆ to C₁₈ aryl. X may be a single bond or substituted or unsubstituted C₆ to C₁₂ aryl.

X may be selected from a single bond or from the following formula 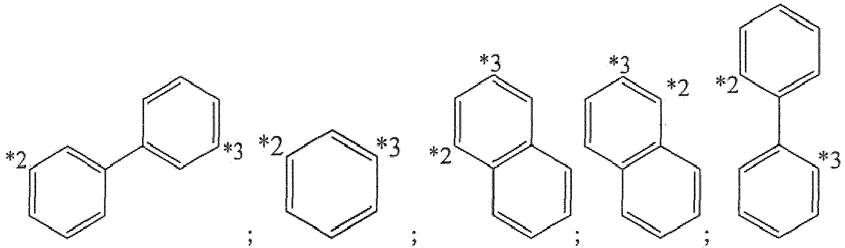 wherein X is bonded to A at ^{∗}2 and is bonded to the ring Z at ^{∗}3.

X may be selected from the structures (Ib) and (Ic) 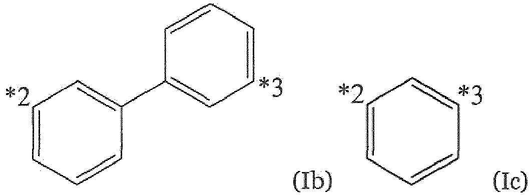 wherein Ib and Ic are bonded to A at ^{∗}2 and are bonded to the ring Z at ^{∗}3.

R is independently selected from the group consisting of substituted or unsubstituted C₁₀ to C₁₈ aryl. R may be independently selected from the group consisting of substituted or unsubstituted C₁₀ to C₁₈ aryl comprising at least two condensed rings and C₁₃ to C₁₈ aryl not comprising condensed rings.

R comprises two 6-membered aryl rings. This applies for both R comprised in the compound of formula (I), respectively.

R is bonded to the (phenylene-)ring Z by a first C-atom of one of the two 6-membered aryl rings; and the first C-atom is adjacent to a second C-atom, wherein the second C-atom is a sp²-C-atom and the second C-atom is not bonded to H. This applies for both R comprised in the compound of formula (I), respectively.

For example, if R has the following structure 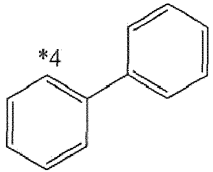 than the carbon atom marked with ^{∗}4^{∗} is the first C-atom and the adjacent carbon atom to which the phenyl-group is bonded is the second C-atom.

In another example, if R has the following structure 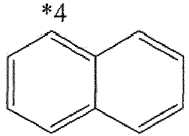 than the carbon atom marked with ^{∗}4^{∗} is the first C-atom and the adjacent bridgehead atom (at which the second ring is condensed) is the second C-atom.

R is independently selected from the group consisting of substituted or unsubstituted C₁₀ to C₁₈ aryl and a substituted or unsubstituted group of the following formula (2) 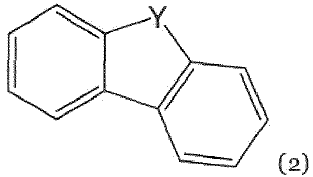 wherein Y is independently selected from the group consisting of CR'₂, SiR'₂, S and O, wherein R' is independently selected from the group consisting of C₁ to C₁₂ alkyl and C₆ to C₁₈ aryl.

R may be independently selected from the group consisting of substituted or unsubstituted C₁₀ to C₁₂ aryl and a substituted or unsubstituted group of the following formula (2) 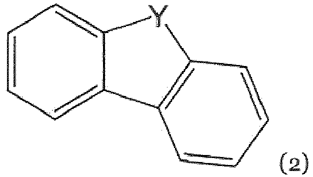 wherein Y is independently selected from the group consisting of CR'₂ and O, wherein R' is independently selected from C₁ to C₄ alkyl, especially methyl.

R may be independently selected from the following groups 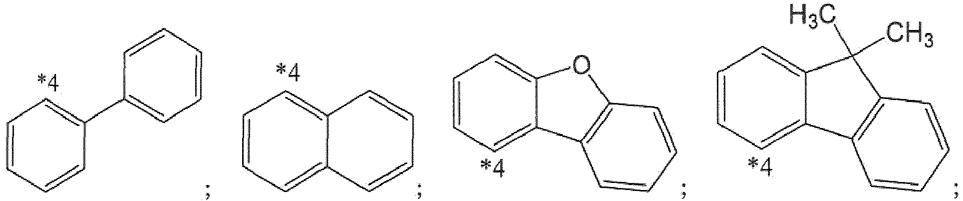 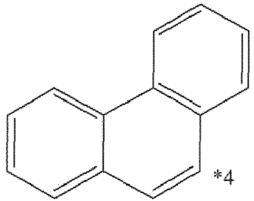 wherein R is bonded the ring Z at ^{∗}4.

R may be independently selected from the following groups 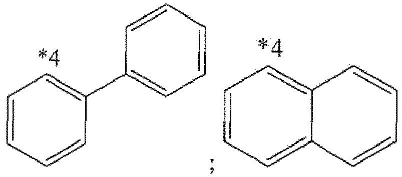 wherein R is bonded the ring Z at ^{∗}4.

X and both R may be selected the same. That is, in such a case, if X is C₆ aryl (phenylene) than both R are likewise C₆ aryl (phenyl).

The compound of formula (II) may have a LUMO energy level in the absolute scale taking vacuum energy level as zero, computed by the TURBOMOLE V6.5 program package using hybrid functional B3LYP and Gaussian 6-31G^{∗} basis set in the range from -1,90 eV to -1.70 eV. The compound of formula (II) may have a LUMO energy level in the absolute scale taking vacuum energy level as zero, computed by the TURBOMOLE V6.5 program package using hybrid functional B3LYP and Gaussian 6-31G^{∗} basis set in the range from -1,85 eV to -1.75 eV. The compound of formula (II) may have a LUMO energy level in the absolute scale taking vacuum energy level as zero, computed by the TURBOMOLE V6.5 program package using hybrid functional B3LYP and Gaussian 6-31G^{∗} basis set in the range from -1,83 eV to -1.78 eV.

The compound of formula (II) may have a refractive index at a wavelength of 633 nm in the range from 1.5 to 2.0, alternatively from 1.6 to 1.9, alternatively from 1.7 to 1.8, such as from 1.738 to 1.763.

The compound of formula (II) may comprise 8 to 13 aromatic or heteroaromatic rings, optionally 8 to 12 aromatic or heteroaromatic rings, optionally 9 to 11 aromatic or heteroaromatic rings.

The compound of formula (II) may comprise 8 to 13 aromatic or heteroaromatic rings, wherein 1 to 3 of the aromatic or heteroaromatic rings are N-containing rings; optionally 8 to 11 aromatic or heteroaromatic rings, wherein 1 to 3 of the aromatic or heteroaromatic rings are N-containing rings; optionally 8 to 12 aromatic or heteroaromatic rings, wherein 1 or 2 of the aromatic or heteroaromatic rings are N-containing rings; and optionally 9 to 11 aromatic or heteroaromatic rings wherein one of the aromatic or heteroaromatic rings is a N-containing rings.

The compound of formula (II) may comprise 8 to 13 aromatic or heteroaromatic rings, wherein 7 to 11 of the aromatic or heteroaromatic rings are aryl rings; optionally 8 to 12 aromatic or heteroaromatic rings, wherein 7 to 10 of the aromatic or heteroaromatic rings are aryl rings; optionally 9 to 11 aromatic or heteroaromatic rings, wherein 8 to 10 of the aromatic or heteroaromatic rings are aryl rings.

The compound of formula (II) may have a ratio of aryl rings to N-containing rings may be from 13:1 to 8:1, such as from 11:1 to 9:1.

The compound of formula (II) may be selected from the following compounds I-1 and I-4 to I-20

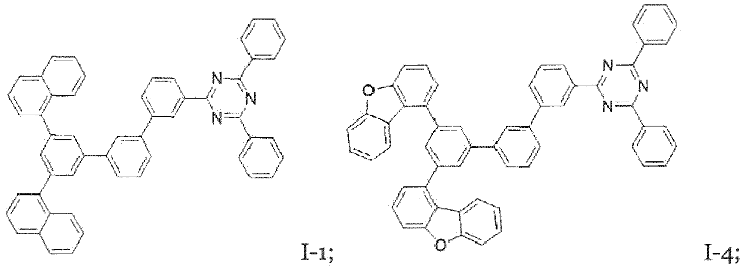

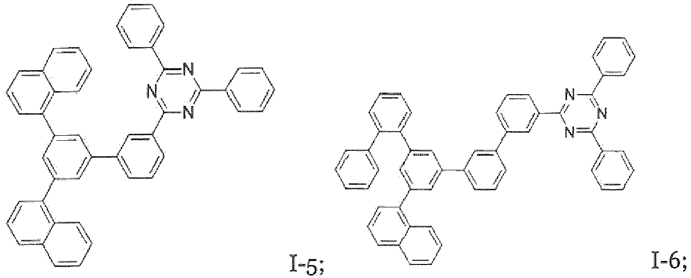

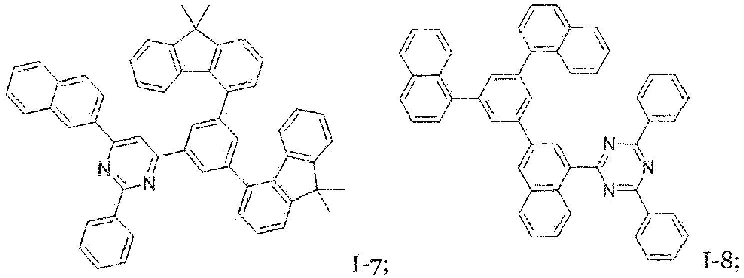

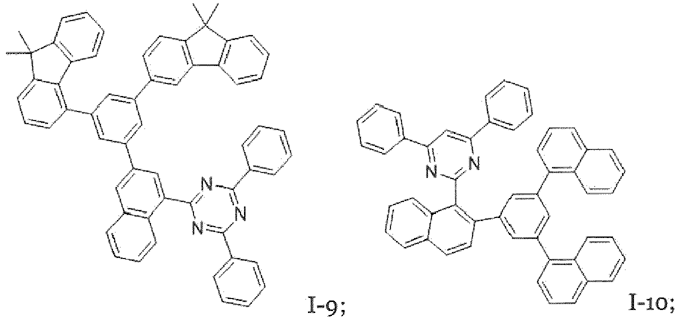

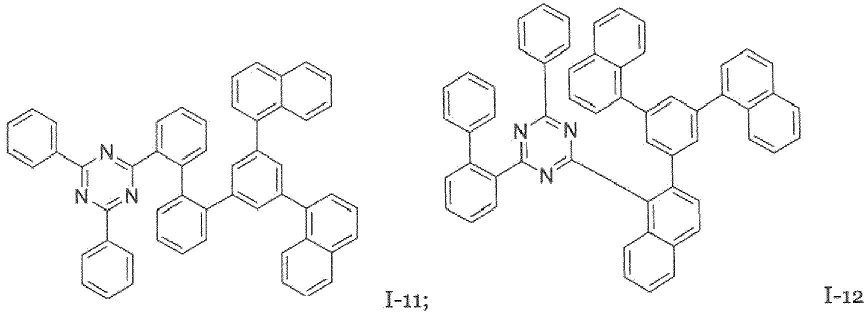

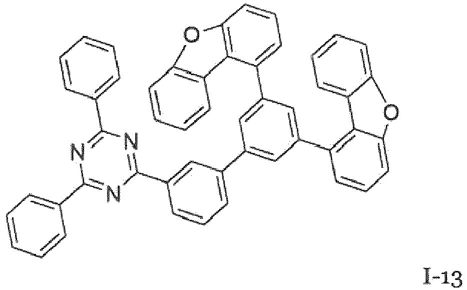

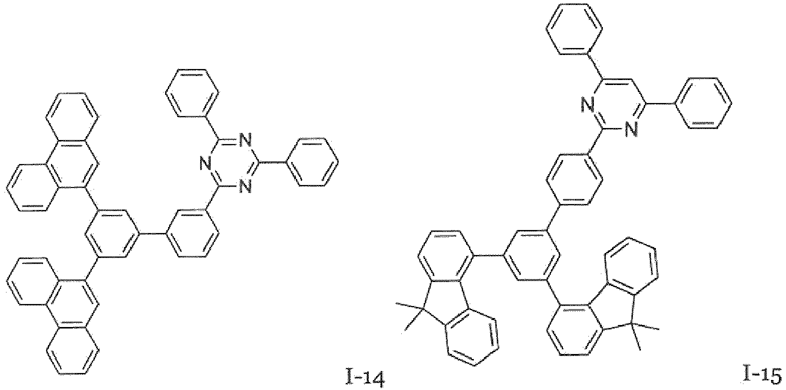

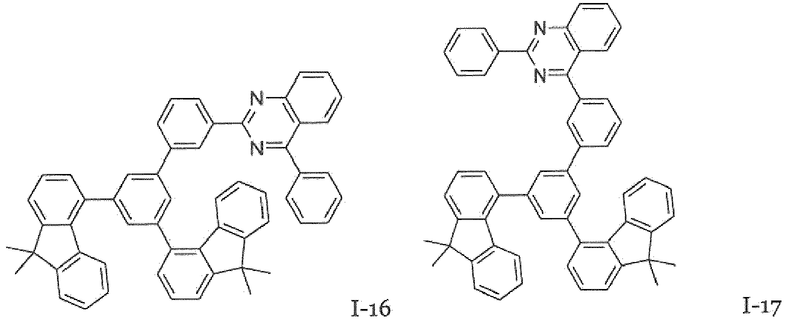

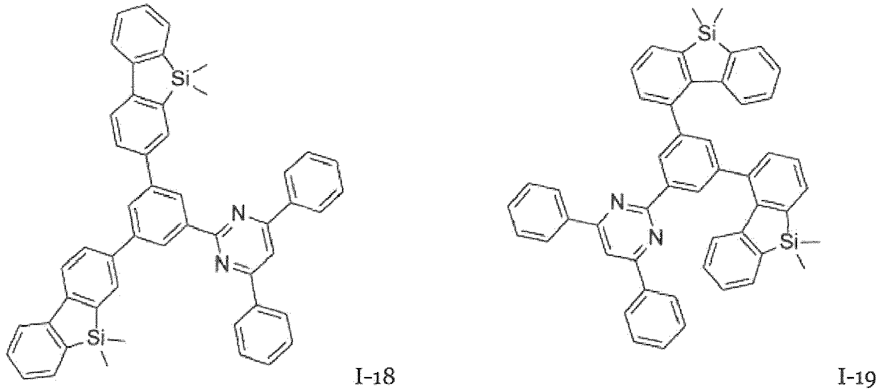

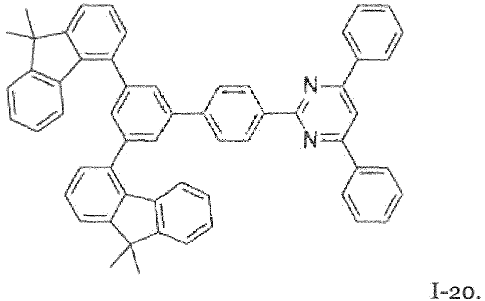

It may be provided that the compound of formula (II) does not comprise a hole transporting moiety.

It may be provided that the compound of formula (II) does not comprise a triarylamine moiety.

It may be provided that the compound of formula (II) does not comprise carbazole.

It may be provided that the following compounds are excluded from formula (II)

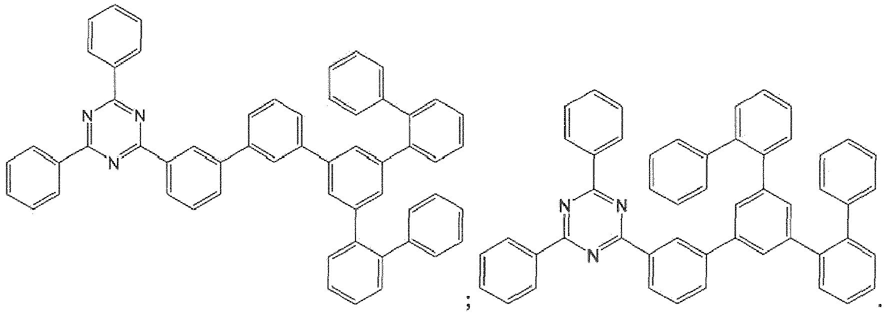

### Exemplary embodiments of the compound of formula (II)

According to one embodiment, there is provided a compound of Formula (II) 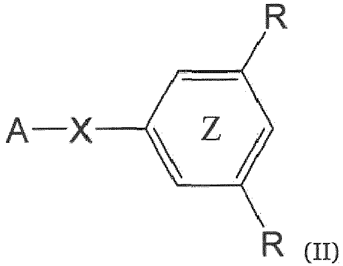 wherein
- A is an unsubstituted or substituted C₃ to C₂₁ heteroaryl comprising at least one six-membered ring, wherein the six-membered ring comprises at least two N-atoms;
   - wherein the substituted C₃ to C₂₁ heteroaryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R is independently selected from the group consisting of substituted or unsubstituted C₁₀ to C₁₈ aryl and a substituted or unsubstituted group of the following formula (2); 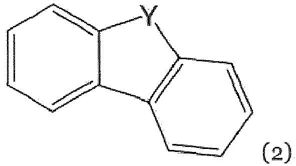
   - wherein Y is independently selected from the group consisting of CR'₂, SiR'2, S and O, wherein R' is independently selected from the group consisting of C₁ to C₁₂ alkyl and C₆ to C₁₈ aryl;
   - wherein the substituted C₁₀ to C₁₈ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
   - wherein the substituted group of the following formula (2) is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R comprises two 6-membered aryl rings;
- R is bonded to the phenylene-ring Z by a first C-atom of one of the two 6-membered aryl rings; and the first C-atom is adjacent to a second C-atom, wherein the second C-atom is a sp²-C-atom and the second C-atom is not bonded to H;
- X is a single bond or substituted or unsubstituted C₆ to C₁₈ aryl;
   - wherein the substituted C₆ to C₁₈ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- X is bonded to a C-atom of the six-membered ring comprising at least two N-atoms of A.

According to one embodiment, there is provided a compound of Formula (II) 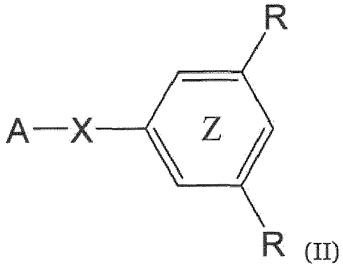 wherein
- A is an unsubstituted or substituted C₃ to C₁₅ heteroaryl comprising at least one six-membered ring, wherein the six-membered ring comprises at least two N-atoms;
   - wherein the substituted C₃ to C₂₁ heteroaryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R is independently selected from the group consisting of substituted or unsubstituted C₁₀ to C₁₂ aryl and a substituted or unsubstituted group of the following formula (2); 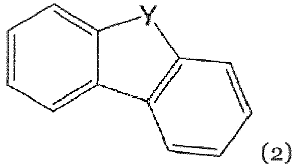
   - wherein Y is independently selected from the group consisting of CR'₂ and O, wherein R' is independently selected from C₁ to C₄ alkyl, especially methyl;
   - wherein the substituted C₁₀ to C₁₂ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
   - wherein the substituted group of the following formula (2) is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R comprises two 6-membered aryl rings;
- R is bonded to the phenylene-ring Z by a first C-atom of one of the two 6-membered aryl rings; and the first C-atom is adjacent to a second C-atom, wherein the second C-atom is a sp²-C-atom and the second C-atom is not bonded to H;
- X is a single bond or substituted or unsubstituted C₆ to C₁₂ aryl;
   - wherein the substituted C₆ to C₁₂ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- X is bonded to a C-atom of the six-membered ring comprising at least two N-atoms of A.

According to one embodiment, there is provided a compound of Formula (II) 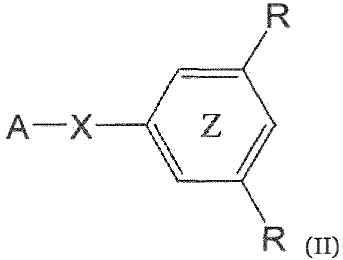 wherein
- A has one of the following formulas 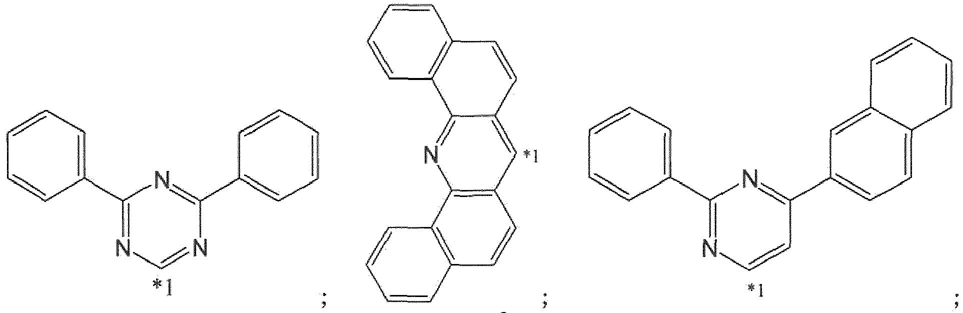 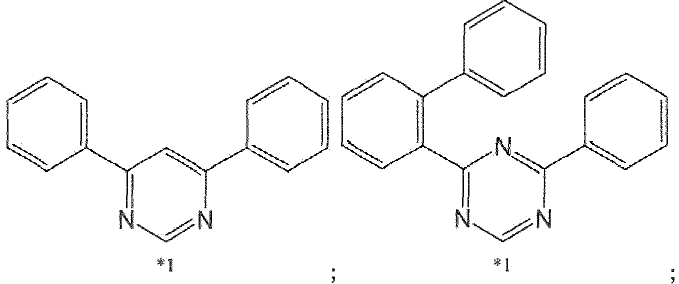 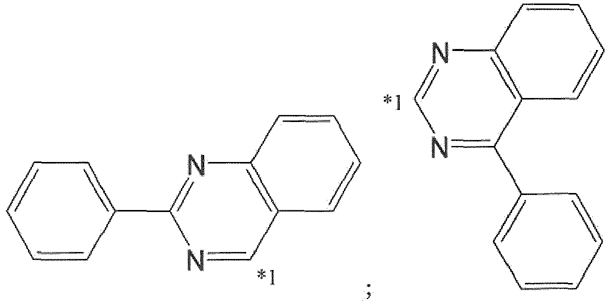 X is selected from a single bond or from the following formulas 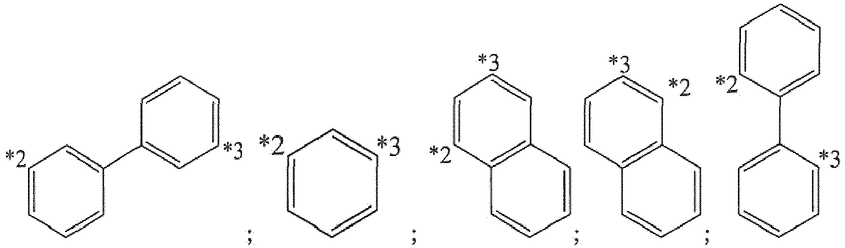
- R is independently selected from the following groups 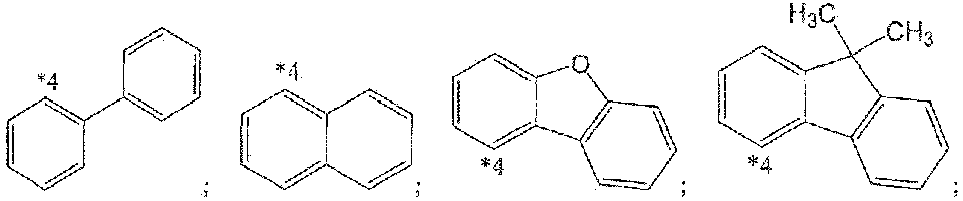 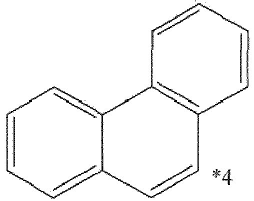 wherein R is bonded the ring Z at ^{∗}4.

### GENERAL DEFINITIONS

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, C₃-alkyl may be selected from n-propyl and iso-propyl. Likewise, C₄-alkyl encompasses n-butyl, sec-butyl and t-butyl. Likewise, C₆-alkyl encompasses n-hexyl and cyclo-hexyl.

If not mentioned else explicitly, all compounds, groups, moieties, substituents etc. shown herein, especially by structural formulas, by systematic names etc. encompass the respective partially and fully deuteriated derivatives thereof.

As used herein if not explicitly mentioned else, the asterisk symbol "^{∗}" represents a binding position at which the moiety labelled accordingly is bond to another moiety.

The subscribed number n in Cₙ relates to the total number of carbon atoms in the respective alkyl, arylene, heteroarylene or aryl group.

The term "aryl" or "arylene" as used herein shall encompass phenyl (C₆-aryl), fused aromatics, such as naphthalene, anthracene, phenanthrene, tetracene etc.. Further encompassed are biphenyl and oligo- or polyphenyls, such as terphenyl, phenyl-substituted biphenyl, phenyl-substituted terphenyl (such as tetraphenyl benzole groups) etc.. "Arylene" respectively "heteroarylene", refers to groups to which two further moieties are attached. In the present specification, the term "aryl group" or "arylene group" may refer to a group comprising at least one hydrocarbon aromatic moiety, and all the elements of the hydrocarbon aromatic moiety may have p-orbitals which form conjugation, for example a phenyl group, a napthyl group, an anthracenyl group, a phenanthrenyl group, a pyrinyl group, a fluorenyl group and the like. Further encompoassed are spiro compounds in which two aromatic moieties are connected with each other via a spiro-atom, such as 9,9'-spirobi[9H-fluorene]yl. The aryl or arylene group may include a monocyclic or fused ring polycyclic (i.e., links sharing adjacent pairs of carbon atoms) functional group.

The term "heteroaryl" as used herein refers to aryl groups in which at least one carbon atom is substituted with a heteroatom. The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably, a heteroarylene ring may comprise at least 1 to 3 heteroatoms individually selected from N, S and/or O. Just as in case of "aryl"/"arylene", the term "heteroaryl" comprises, for example, spiro compounds in which two aromatic moieties are connected with each other, such as spiro[fluorene-9,9'-xanthene]. Further exemplary heteroaryl groups are diazine, triazine, dibenzofurane, dibenzothiofurane, acridine, benzoacridine, dibenzoacridine etc.

The term "alkenyl" as used herein refers to a group -CR¹ = CR²R³ comprising a carbon-carbon double bond.

The term "perhalogenated" as used herein refers to a hydrocarbyl group wherein all of the hydrogen atoms of the hydrocarbyl group are replaced by halogen (F, Cl, Br, I) atoms.

The term "alkoxy" as used herein refers to a structural fragment of the Formula -OR with R being hydrocarbyl, preferably alkyl or cycloalkyl.

The term "thioalkyl" as used herein refers to a structural fragment of the Formula -SR with R being hydrocarbyl, preferably alkyl or cycloalkyl.

The subscripted number n in Cₙ-heteroaryl merely refers to the number of carbon atoms excluding the number of heteroatoms. In this context, it is clear that a C₃ heteroarylene group is an aromatic compound comprising three carbon atoms, such as pyrazol, imidazole, oxazole, thiazole and the like.

The term "heteroaryl" as used herewith shall encompass pyridine, quinoline, benzoquinoline, quinazoline, benzoquinazoline, pyrimidine, pyrazine, triazine, benzimidazole, benzothiazole, benzo[4,5]thieno[3,2-d]pyrimidine, carbazole, xanthene, phenoxazine, benzoacridine, dibenzoacridine and the like.

In the present specification, the term single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In accordance with the present disclosure, in a formula showing the following binding situation, 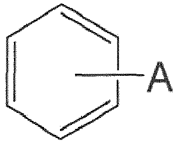 the group A may be bound to any suitable binding position. In a situation were it is shown that the bond of A crosses more than one ring 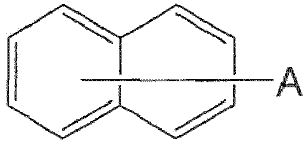 the group A may be bound to any suitable binding position of each ring crossed with the bond.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

The term "contacting sandwiched" refers to an arrangement of three layers whereby the layer in the middle is in direct contact with the two adjacent layers.

With respect to the inventive electron transport layer stack the compounds mentioned in the experimental part are most preferred.

A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

According to another aspect, the organic electroluminescent device according to the present invention comprises two or three or more emission layers. An OLED comprising more than one emission layer is also described as a tandem OLED or stacked OLED.

The organic electroluminescent device (OLED) may be a bottom- or top-emission device. The organic electroluminescent device (OLED) may emit the light trough a transparent anode or through a transparent cathode.

Another aspect is directed to a device comprising at least one organic electroluminescent device (OLED).

A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula.

The terms "OLED" and "organic light-emitting diode" are simultaneously used and have the same meaning. The term "organic electroluminescent device" as used herein may comprise both organic light emitting diodes as well as organic light emitting transistors (OLETs).

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of', "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Preferably, the first electron transport layer comprising the compound of Formula (I) and the second electron transport layer are essentially non-emissive or non-emitting.

The operating voltage, also named U, is measured in Volt (V) at 10 milliAmpere per square centimeter (mA/cm²).

The candela per Ampere efficiency, also named cd/A efficiency is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color. Efficiency values are compared at the same CIE-y.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

The anode and cathode may be described as anode electrode / cathode electrode or anode electrode / cathode electrode or anode electrode layer / cathode electrode layer.

Room temperature, also named ambient temperature, is 23° C.

Hereinafter, the embodiments are illustrated in more detail with reference to examples. However, the present disclosure is not limited to the following examples. Reference will now be made in detail to the exemplary aspects.

### DESCRIPTION OF THE DRAWINGS

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
FIG. 1 is a schematic sectional view of an OLED, according to an exemplary embodiment of the present invention.
FIG. 2 is a schematic sectional view of an OLED comprising a charge generation layer and two emission layers, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

Fig. 1 is a schematic sectional view of an OLED 100, according to an exemplary embodiment of the present invention. The OLED 100 of Fig. 1 comprises an electron blocking layer (EBL) 145 and the first electron transport layer (= hole blocking layer; HBL) 155.

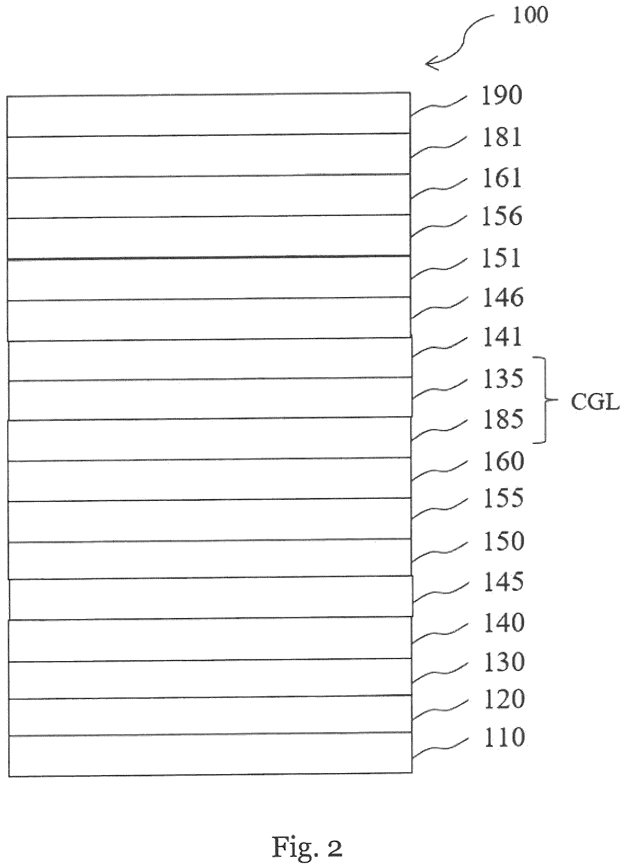

Referring to Fig. 2, the OLED 100 includes a substrate 110, an anode 120, a hole injection layer (HIL) 130, a hole transport layer (HTL) 140, an electron blocking layer (EBL) 145, an emission layer (EML) 150, the first electron transport layer 155 comprising the compound of formula (I), the second electron transport layer, an electron injection layer (EIL) 180 and a cathode electrode 190.

Fig. 2 is a schematic sectional view of an OLED 100, according to another exemplary embodiment of the present invention. Fig. 2 differs from Fig. 1 in that the OLED 100 of Fig. 2 further comprises a charge generation layer (CGL) and a second emission layer (151).

Referring to Fig. 2, the OLED 100 includes a substrate 110, an anode 120, a first hole injection layer (HIL) 130, a first hole transport layer (HTL) 140, a first electron blocking layer (EBL) 145, a first emission layer (EML) 150, a first electron transport layer comprising the compound of formula (I) (HBL) 155, a second electron transport layer 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a further first electron transport layer 156 comprising the compound of formula (I), a further second electron transport layer 161, a second electron injection layer (EIL) 181 and a cathode 190.

While not shown in Fig. 1 and Fig. 2, a sealing layer may further be formed on the cathode electrodes 190, in order to seal the OLEDs 100. In addition, various other modifications may be applied thereto.

Hereinafter, the embodiments are illustrated in more detail with reference to examples. However, the present disclosure is not limited to the following examples.

### DETAILED DESCRIPTION

The invention is furthermore illustrated by the following examples which are illustrative only and non-binding.

### Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 µL Mettler Toledo aluminum pan with lid, a <1 mm hole is pierced into the lid).

### Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

### Rate onset temperature

The rate onset temperature (TRO) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than 10-5 mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature. To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

### Reduction potential

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard Fc⁺/Fc redox couple, afforded finally the values reported above. All studied compounds as well as the reported comparative compounds showed well-defined reversible electrochemical behaviour.

### Dipole moment

The dipole moment |*̅µ̅*̅| of a molecule containing N atoms is given by: 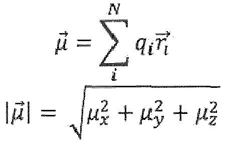 where *qᵢ* and *̅r̅ₗ̅*̅ are the partial charge and position of atom i in the molecule.

The dipole moment is determined by a semi-empirical molecular orbital method.

The geometries of the molecular structures are optimized using the hybrid functional B3LYP with the 6-31G^{∗} basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

### Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G^{∗} basis set in the gas phase. If more than one conformation is viable, the conformation with the lowest total energy is selected.

### Physical properties

HOMO, LUMO and Dipole moment values for the inventive compounds and the comparative compound ET-3 are summarized in the following Table 1.

**Table 1:**

| Compound name | HOMO [eV] | LUMO [eV] | Dipole [Debye] |
|---|---|---|---|
| I-1 | -5,66 | -1,78 | 0,39 |
| I-2 | -5,63 | -1,74 | 1,76 |
| I-3 | -5,81 | -1,83 | 0,10 |
| I-4 | -5,89 | -1,85 | 0,94 |
| I-5 | -5,67 | -1,84 | 0,04 |
| I-6 | -5,64 | -1,81 | 0,26 |
| I-7 | -5,71 | -1,73 | 2,63 |
| I-8 | -5,65 | -1,90 | 0,19 |
| I-9 | -5,66 | -1,88 | 0,85 |
| I-10 | -5,57 | -1,66 | 2,18 |
| I-11 | -5,54 | -1,78 | 0,47 |
| I-12 | -5,61 | -1,73 | 0,37 |
| I-13 | -5,92 | -1,84 | 0,62 |
| I-14 | -5,65 | -1,82 | 0,34 |
| ET-3 | -5,98 | -1,80 | 0,24 |

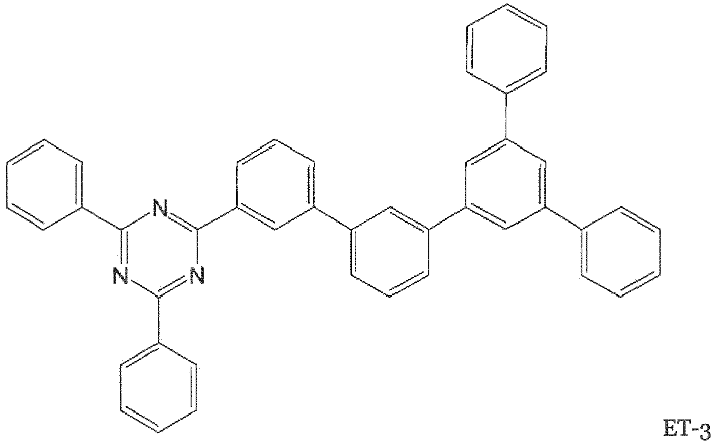

### Synthesis Procedures

### Compound I-1: 2-(3",5"-di(naphthalen-1-yl)-[1,1':3',1"-terphenyl]-3-yl)-4,6-diphetiyl-1,3,5-triazine

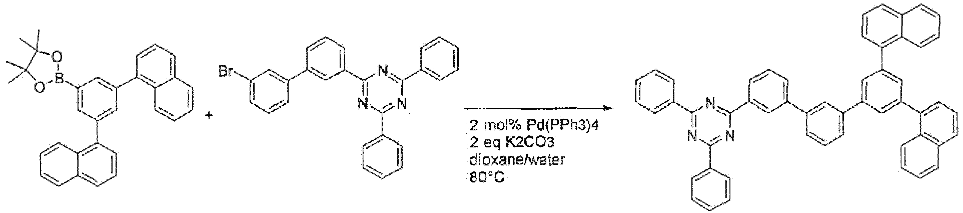

3 Neck round-bottom flask was flushed with nitrogen and charged with 2-(3,5-di-1-naphthalenylphenyl)-4,4,5,5-tetramethyl-1,3,2-dioxaborolane (CAS 957107-94-7) 1,05 eq (31,0 g), 2-(3'-bromo-[1,1'-biphenyl]-3-yl)-4,6-diphenyl-1,3,5-triazine (CAS 1606981-69-4) 1 eq (30,0 g), tetrakis(triphenylphosphine)palladium(o) (CAS 14221-01-3) 0,02 eq (1,6 g), potassium carbonate (CAS 584-08-7) 2 eq (18,5 g). Deaerated mixture of 402 ml dioxane and 67 ml water was added. Reaction was running at 80°C under a nitrogen atmosphere overnight. Reaction was cooled down and solvent was evaporated. Raw product was extracted in 400 ml chloroform/water. Combined organic phases were dried and filtered through a silica pad. Final purification was done by sublimation. Yield: 32,4 g (67%). (ESI-APCI-MS:713).

### Compound I-14: 2-(3',5'-di(phenanthren-9-yl)-[1,1'-biphenyl]-3-yl)-4,6-diphenyl-1,3,5-triazine

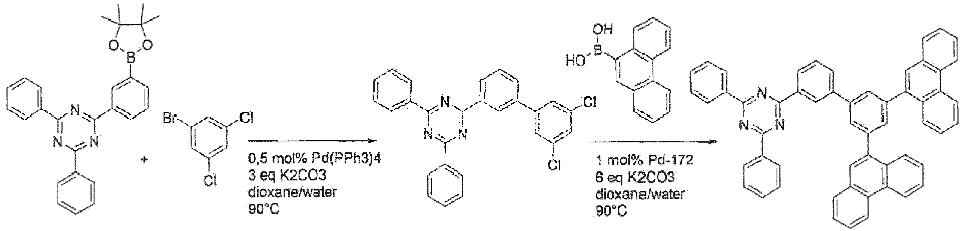

### 1. Step. 2-(3',5'-dichloro-[1,1'-biphenyl]-3-yl)-4,6-diphenyl-1,3,5-triazine.

3 Neck round-bottom flask was flushed with nitrogen and charged with 2,4-diphenyl-6-(3-(4,4,5,5-tetramethyl-1,3,2-dioxaborolan-2-yl)phenyl)-1,3,5-triazine (CAS 1269508-31-7) 1 eq (10,0 g), 1-bromo-3,5-dichlorobenzene (CAS 19752-55-7) 1 eq (5,2 g), tetrakis(triphenylphosphine)palladium(o) (CAS 14221-01-3) 0,005 eq (0,13 g), potassium carbonate (CAS 584-08-7) 3 eq (9,5 g). Deaerated mixture of 150 ml dioxane and 38 ml water was added. Reaction was running at 90°C under a nitrogen atmosphere overnight. A white suspension was formed. Reaction was cooled down, raw product was filtered, washed with water and cold dioxane. White solid was then dissolved in 400 ml chloroform and filtered through a silica pad. Resulting solution was evaporated at low pressure, pale-white solid was recrystallized from dioxane. Yield: 7,8 g (73%). (ESI-APCI-MS:454).

### 2. Step. 2-(3',5'-di(phenanthren-9-yl)-[1,1'-biphenyl]-3-yl)-4,6-diphenyl-1,3,5-triazine

3 Neck round-bottom flask was flushed with nitrogen and charged with 2-(3',5'-dichloro-[1,1'-biphenyl]-3-yl)-4,6-diphenyl-1,3,5-triazine (no CAS) 1 eq (15,0 g), phenanthren-9-ylboronic acid (CAS 68572-87-2) 2,6 eq (19,1 g), chloro(crotyl)(2-dicyclohexylphosphino-2',6'-dimethoxybiphenyl)palladium(II) (CAS 1798781-99-3) 0,01 eq (0,2 g), potassium carbonate (CAS 584-08-7) 6 eq (27,4 g). Deaerated mixture of 450 ml dioxane and 120 ml water was added. Reaction was running at 90°C under a nitrogen atmosphere for 3 days. A thick suspension was formed. Reaction mixture was cooled down, raw product was filtered, washed with water, cold dioxane and hexane. Greyish solid was put in Soxhlet apparatus and extracted with toluene overnight. Resulting suspension was filtered, washed with toluene and hexane, dried to give a white crystalline solid. Final purification was done by sublimation. Yield: 16,8 g (69%). (ESI-APCI-MS:738).

### Compound I-13: 2-(3',5'-Bis(dibenzo[b,d]furan-1-yl)-[1,1'-biphenyl]-3-yl)-4,6-diphenyl-1,3,5-triazine

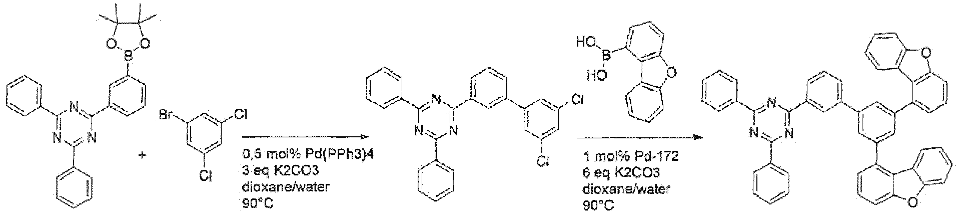

### 1. Step. 2-(3',5'-dichloro-[1,1'-biphenyl]-3-yl)-4,6-diphenyl-1,3,5-triaxine

### As described above.

### 2. Step. 2-(3',5'-Bis(dibenzo[b,d]furan-1-yl)-[1,1'-biphenyl]-3-yl)-4,6-diphenyl-1,3,5-triazine

3 Neck round-bottom flask was flushed with nitrogen and charged with 2-(3',5'-dichloro-[1,1'-biphenyl]-3-yl)-4,6-diphenyl-1,3,5-triazine (no CAS) 1 eq (15,0 g), dibenzo[b,d]furan-1-ylboronic acid (CAS 162607-19-4) 2,6 eq (18,2 g), chloro(crotyl)(2-dicyclohexylphosphino-2',6'-dimethoxybiphenyl)palladium(II) (CAS 1798781-99-3) 0,01 eq (0,2 g), potassium carbonate (CAS 584-08-7) 6 eq (27,4 g). Deaerated mixture of 450 ml dioxane and 120 ml water was added. Reaction was running at 90°C under a nitrogen atmosphere for 3 days. A thick suspension was formed. Reaction mixture was cooled down, raw product was filtered, washed with water, cold dioxane and hexane. Greyish solid was put in Soxhlet apparatus and extracted with toluene overnight. Resulting suspension was cooled down, filtered, washed with toluene and hexane, dried to give a white crystalline solid. The material was additionally recrystallized from toluene. Final purification was done by sublimation. Yield: 15,5 g (87%). (ESI-APCI-MS:718).

### General procedure for fabrication of OLEDs

For bottom emission OLED device, an ITO/glass substrate was cut to a size of 150 mm x 150 mm x 0.7 mm, rinse with isopropyl alcohol for ~5 minutes and then with ultrasonically cleaned pure water for~ 5 minutes, bake out in oven at 200°C for ~2h.

### Before organic material deposition it gets plasma treatment in vacuum chamber (typically N2 plasma)

Then, HT-1 and D-1 were vacuum co-deposited on the ITO to form a HIL. Then, HT-1 was vacuum deposited on the HIL to form an HTL. Then, HT-2 was vacuum deposited on the HTL to form an electron blocking layer (EBL).

Afterwards the emission layer was deposited by co-deposition of HOST-1:EMITTER-1.

Afterwards, a compound of Formula I-1, I-3 or ET-3 was vacuum deposited onto the emission layer to form the hole blocking layer, respectively. Then, the electron transporting layer is formed on the hole blocking layer by co-deposition ET-1 and lithium quinolate (LiQ).

Then, Al is evaporated at a rate of ~3 Å/s at 10-7 mbar to form a cathode with a thickness of 100 nm.

The details of the layer stack in the bottom emission OLED devices are given below. A slash "/" separates individual layers. Layer thicknesses are given in squared brackets [...], mixing ratios in round brackets (...):

### Layer stack details used in the OLED device examples of Table 3:

ITO [90 nm] / HT-1:D-1 (wt% 98:2) [10nm] / HT-1 [128nm] / HT-2 [5nm] / HOST-1:EMITTER-1 (vol% 97:3) [20 nm] / Compound I-1, I-3 or ET-3 [5nm] / ET-1:LiQ (vol% 50:50) [31nm] / Al [100nm]

**Table 2: List of compounds used**

| | IUPAC name | Reference |
|---|---|---|
| HT-1 | N-([1,1'-biphenyl]-4-yl)-9,9-dimethyl-N-(4-(9-phenyl-9H-carbazol-3-yl)phenyl)-9H-fluoren-2-amine CAS 1242056-42-3 | US2016322581 |
| HT-2 | N-([1,1'-biphenyl]-4-yl)-9,9-diphenyl-N-(4-(triphenylsilyl)phenyl)-9H-fluoren-2-amine [CAS 1613079-70-1] | WO2014088047 |
| D-1 | 4,4',4"-((1E,1'E,1"E)-cyclopropane-1,2,3-triylidenetris(cyanomethanylylidene))tris(2,3,5,6-tetrafluorobenzonitrile) [CAS 1224447-88-4] | US2008265216 |
| HOST-i | H09 (Fluorescent-blue host material) | Commercially available from Sun Fine Chemicals, Inc, S. Korea |
| EMITTER-1 | BD200 (Fluorescent-blue emitter material) | Commercially available from Sun Fine Chemicals, Inc, S. Korea |
| ET-1 | 2-(2',6'-diphenyl-[1,1':4',1"-terpheny]-4-yl)-4-phenyl-6-(3-(pyridin-4-yl)phenyl)-1,3,5-triazine | EP3923364 A1 |
| ET-3 | 2,4-diphenyl-6-(5"-phenyl-[1,1':3',1":3",1‴-quaterphenyl]-3-yl)-1,3,5-triazine | KR 1948709 B1 |
| | CAS 1802231-36-2 | |
| LiQ | 8-Hydroxyquinolinolato-lithium | WO2013079217 |
| | [CAS 850918-68-2] | |

### OLED data

**Table 3: OLED device lifetime and voltage rise of OLED devices (ralatice to comparative example)**

| **OLED device examples** | **HBL** | **LT97 @ 30mA/cm²** | **Voltage rise, 1-50h** |
|---|---|---|---|
| Comparative example-1 | ET-3 | 100% | 100% |
| Example-1 | I-3 | **124%** | **55%** |
| Example-2 | I-1 | **161%** | **76%** |

### Technical Effect of the invention

The OLED devices according to the invention comprising an electron transport layer comprising a compound of formula (I) show **improved lifetime (durability)** and improved (decreased) voltage rise over time over the comparative device.

The features disclosed in the foregoing description and in the dependent claims may, both separately and in any combination thereof, be material for realizing the aspects of the disclosure made in the independent claims, in diverse forms thereof.

## Claims

1. Organic light emitting diode comprising a non-transparent substrate, an anode, a cathode, an emission layer, an electron injection layer and an electron transport layer stack;
wherein
- the electron transport layer stack is arranged between the emission layer and the electron injection layer;
- the electron transport layer stack comprises a first electron transport layer and a second electron transport layer;
- the first electron transport layer is in direct contact with the emission layer;
- the first electron transport layer comprises a compound of formula (I) 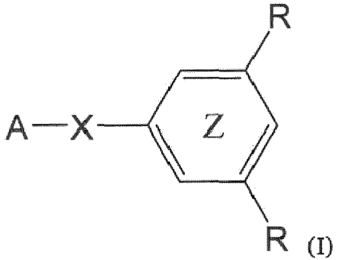 wherein
- A is an unsubstituted or substituted C₃ to C₂₁ heteroaryl comprising at least one six-membered ring, wherein the six-membered ring comprises at least one N-atom;
- wherein the substituted C₃ to C₂₁ heteroaryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R is independently selected from the group consisting of substituted or unsubstituted C₁₀ to C₁₈ aryl and a substituted or unsubstituted group of the following formula (2); 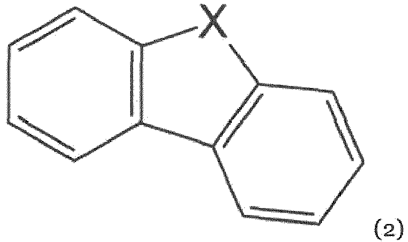
- wherein X is independently selected from the group consisting of CR'₂, SiR'2, S and O, wherein R' is independently selected from the group consisting of C₁ to C₁₂ alkyl and C₆ to C₁₈ aryl;
- wherein the substituted C₁₀ to C₁₈ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C, to C₇ alkyl, perfluorinated C₁ to C₇ alkyl, partially fluorinated C₁ to C₇ alkoxy, perfluorinated C₁ to C₇ alkoxy, D, F and CN;
- wherein the substituted group of the following formula (2) is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R comprises two 6-membered aryl rings;
- R is bonded to the ring Z by a first C-atom of one of the two 6-membered aryl rings; and the first C-atom is adjacent to a second C-atom, wherein the second C-atom is a sp²-C-atom and the second C-atom is not bonded to H;
- X is a single bond or substituted or unsubstituted C₆ to C₆₀ aryl;
- wherein the substituted C₆ to C₆₀ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- X is bonded to a C-atom of the six-membered ring comprising at least one N-atom of A; and
- the second electron transport layer does not comprise a compound according to formula (I).

2. Organic light emitting diode according to claim 1, wherein A is a substituted C₃ to C₂₁ heteroaryl comprising at least one six-membered ring, wherein the six-membered ring comprises at least two N-atoms;
- wherein the substituted C₃ to C₂₁ heteroaryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN.

3. Organic light emitting diode according to claim 1 or 2, wherein A has the following formula (Ia) 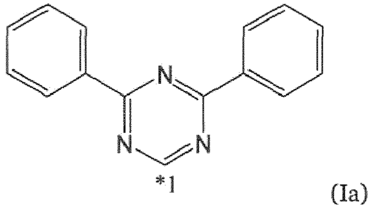 wherein Ia is bonded at *1 to X.

4. Organic light emitting diode according to any of the preceding claims, wherein X is C₆ to C₁₈ arylene.

5. Organic light emitting diode according to any of the preceding claims, wherein X selected from the structures (Ib) and (Ic) 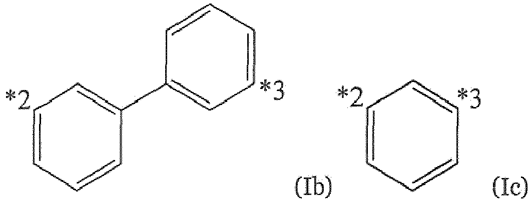 wherein Ib and Ic are bonded to A at ^{∗}2 and are bonded to the ring Z at ^{∗}3.

6. Organic light emitting diode according to any of the preceding claims, wherein R is independently selected from the group consisting of substituted or unsubstituted C₁₀ to C₁₂ aryl;
- wherein the substituted C₁₀ to C₁₂ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;

7. Organic light emitting diode according to any of the preceding claims, wherein R is independently selected from the structures (Id) and (Ie) 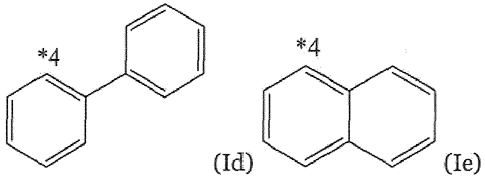 wherein Id and Ie are bonded the ring Z at ^{∗}4.

8. Organic light emitting diode according to any of the preceding claims, wherein the first electron transport layer consists of the compound of Formula (I).

9. Organic light emitting diode according to any of the preceding claims, wherein X and both R are selected the same.

10. Display device comprising the organic light emitting diode according to any of the preceding claims.

11. Compound of Formula (II) 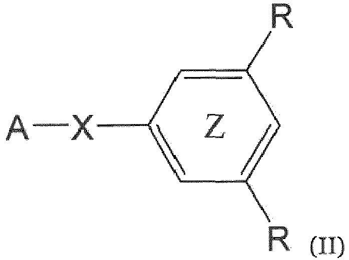 wherein
- A is an unsubstituted or substituted C₃ to C₂₁ heteroaryl comprising at least one six-membered ring, wherein the six-membered ring comprises at least two N-atoms;
- wherein the substituted C₃ to C₂₁ heteroaryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R is independently selected from the group consisting of substituted or unsubstituted C₁₀ to C₁₈ aryl and a substituted or unsubstituted group of the following formula (2) 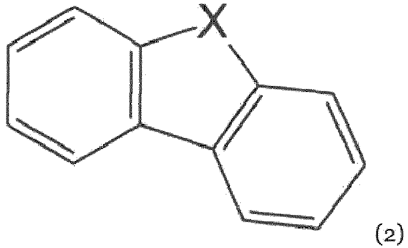
- wherein X is independently selected from the group consisting of CR'₂, SiR'₂, S and O, wherein R' is independently selected from the group consisting of C₁ to C₁₂ alkyl and C₆ to C₁₈ aryl;
- wherein the substituted C₁₀ to C₁₈ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- wherein the substituted group of the following formula (2) is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- R comprises two 6-membered aryl rings;
- R is bonded to the phenylene-ring Z by a first C-atom of one of the two 6-membered aryl rings; and the first C-atom is adjacent to a second C-atom, wherein the second C-atom is an sp²-C-atom and the second C-atom is not bonded to H;
- X is a single bond or C₆ to C₆₀ aryl;
- wherein the substituted C₆ to C₆₀ aryl is substituted with one or more substituents independently selected from the group consisting of C₁ to C₇ alkyl, C₁ to C₇ alkoxy, partially deuterated C₁ to C₇ alkyl, perdeuterated C₁ to C₇ alkyl, partially deuterated C₁ to C₇ alkoxy, perdeuterated C₁ to C₇ alkoxy, partially fluorinated C₁ to C₇ alkyl, perfluorinated C₁ to C₇ alkyl, partially fluorinated C₁ to C₇ alkoxy, perfluorinated C₁ to C₇ alkoxy, D, F and CN;
- X is bonded to a C-atom of the six-membered ring comprising at least two N-atoms of A.
